# EUROPEAN PATENT SPECIFICATION

(11) **EP 3 603 611 B1**
(45) Date of publication and mention of the grant of the patent: **10.01.2024**
(21) Application number: 18770447.3
(22) Date of filing: 09.03.2018
(51) Int. Cl.: A61K 8/44, A61Q 1/04, A61Q 1/12, A61Q 1/14, A61Q 17/04, A61Q 19/02, A61Q 19/10, G01N 30/72, G01N 30/88, A61K 8/49

(54) **SKIN WHITENING AGENT COMPRISING SPECIFIC D-AMINO ACIDS**
HAUTAUFHELLENDES MITTEL ENTHALTEND SPEZIFISCHEN D-AMINOSÄUREN
AGENT DE BLANCHIMENT DE LA PEAU COMPRENANT DES ACIDES AMINÉS D SPÉCIFIQUES

(30) Priority: 24.03.2017 JP 2017060070
(43) Date of publication of application: 05.02.2020
(73) Proprietor: Ajinomoto Co., Inc., Tokyo 104-8315 (JP)
(72) Inventor: TAKINO, Yoshinobu, Kawasaki-shi Kanagawa 210-8681 (JP); KARAKAWA, Sachise, Kawasaki-shi Kanagawa 210-8681 (JP); OKURA, Fumie, Kawasaki-shi Kanagawa 210-8681 (JP); IKEGAMI, Eri, Kawasaki-shi Kanagawa 210-8681 (JP)
(74) Representative: Mewburn Ellis LLP
(86) International application number: PCT/JP2018/009351
(87) International publication number: WO 2018/173816

(56) References cited:
- EP-A2- 0 728 469
- JP-A- H1 149 629
- JP-A- H1 149 630
- JP-A- H10 158 149
- JP-A- 2004 315 384
- JP-A- 2005 247 738
- JP-A- 2008 088 113
- JP-A- 2008 088 113
- JP-A- 2012 006 870
- JP-A- 2012 041 285
- US-A1- 2013 079 410
- TOJO, YOSUKE et al.: "Free D-amino acids as physiologically active molecules in skin tissue", FRAGRANCE JOURNAL, April 2016 (2016-04), pages 14-18, XP9516317,
- ASHIDA, YUTAKA et al.: "Presence of free D-amino acids in the skin and their physiological functions", BIO INDUSTRY, vol. 28, no. 2, 2011, pages 40-44, XP9516313,
- MIKAMI, NAOKO: "Amino acid and beatification", BIO INDUSTRY, vol. 25, no. 10, 2008, pages 21-25, XP9516314,

## Description

### Field

The present invention relates to a whitening agent.

### Background

Pigmented spot, freckles, and somberness of the skin occur by the production and deposition of a black melanin pigment from pigment cells (melanocytes) caused by irradiation with ultraviolet rays and the like. It is known that the melanin pigment is produced by tyrosinase and tyrosinase-related protein with tyrosine as an amino acid substance as a starting substance. Given these circumstances, to inhibit the deposition of melanin, a technique having an action of inhibiting tyrosinase and tyrosinase-related protein 1 (TRP-1) is presented (Patent Literature 1).

For the purpose of preventing or reducing inflammation of the skin induced by ultraviolet rays and deterioration of the skin associated therewith through single intake using an extract derived from a plant whose edibility is known, an oral skin protective agent containing crocetin, one of carotenoid pigments, is presented (Patent Literature 2).

D-Alanine and D-hydroxyproline are reported as D-amino acids having an influence on a protein present in a large amount in a basement membrane that separates epidermis and dermis (Patent Literature 3).

Disclosed is a skin external preparation containing a whitening agent selected from a hydroquinone glycoside, alkoxy salicylic acid and/or a salt thereof, and α-glycosyl-L-ascorbic acid, one or two or more betaine derivatives, a higher fatty acid, and one or two or more selected from L-serine, D-serine, DL-serine, alanine, and aminomethyl propanediol for the purpose of markedly improving a whitening effect and an action of improving skin roughness (Patent Literature 4).

Patent literature 5 describes skin-brightening compositions containing leucine or isoleucine as the skin-brightening active ingredient.

Patent literature 6 describes an endermic liniment containing tyrosine derivatives.

### Citation List

### Patent Literature

Patent Literature 1: WO 2013/118887
Patent Literature 2: Japanese Patent Application Laid-open No. 2013-67592
Patent Literature 3: WO 2011/040082
Patent Literature 4: Japanese Patent Application Laid-open No. 2002-060313
Patent Literature 5: Japanese Patent Application No. 2008-088113 A
Patent Literature 6: EP 0 728 469 A2

### Summary

### Technical Problem

A bioactive substance having an action of inhibiting tyrosinase disclosed in Patent Literature 1 is obtained by alcohol-extracting Cacalia hastata ssp. orientalis and/or Parasenecio hastatus ssp. tanakae. However, it is disclosed that a melanin pigment is inhibited by a tyrosinase inhibiting component and an antioxidant component in addition to a microphthalmia-associated transcription factor (MITF) inhibiting action. That is to say, it is recognized that the bioactive substance having an action of inhibiting tyrosinase disclosed in Patent Literature 1 alone is insufficient in an action of inhibiting tyrosinase gene expression.

Crocetin disclosed in Patent Literature 2 is disclosed as having an effect on erythema intensity (that is, inflammation by ultraviolet rays) in its Example. However, there is no demonstration that the effect is obtained even in a state in which melanin deposits such as pigmented spot and freckles.

Patent Literature 3 discloses that a laminin 332 production promoting effect is not expressed when a D-amino acid compound such as D-aspartic acid is used. In addition, it discloses laminin 332 production promotion in the skin and does not describe nor suggest a technique that inhibits the production of proteins other than laminin 332.

The skin external preparation described in Patent Literature 4 contains a specific whitening agent different from D-Ser as an essential component, and it does not describe nor suggest a whitening action of D-Ser itself.

The present invention has been made in view of the above problems, and an object thereof is to provide a whitening agent that can sufficiently inhibit the production of melanin production-related proteins and exhibit a sufficient whitening effect.

### Solution to Problem

The present inventors diligently have studied to achieve the above object. As a result, the present inventors have found that there is a correlation between skin conditions (for example, melanin index) of a subject and specific D-amino acids contained in a horny layer.

Based on such findings, the present inventors have thought that the specific D-amino acids can suppress a production of a melanin production-related protein such as tyrosinase, tyrosinase-related protein 1, and melanocortin 1 receptor, and reached the present invention.

That is, the present invention is as follows.

Non-therapeutic use of a composition according to claim 1 as a skin-whitening agent. Non-therapeutic use of a composition according to claim 5 in a method of prevention, inhibition, or treatment of pigmented spot, freckles, or somberness.

A method of evaluation of a skin by a D-amino acid according to claim 6.

More specific embodiments are defined in the dependent claims.

In the present specification, the amino acids may be represented by a conventional three-letter notation (for example, "Asn", "Gln") with a configuration prefix such as "L-", "D-", "D,L-" or the like.

### Advantageous Effects of Invention

The composition used in the present invention has a sufficient whitening effect.

### Brief Description of Drawings

FIG. 1 is a diagram of a correlation between a D-Pro amount in a horny layer and a melanin index of a skin in women in their twenties or thirties.
FIG. 2 is a diagram of a correlation between a D-His amount in the horny layer and the melanin index of the skin in women in their forties or fifties.
FIG. 3 is a diagram of a correlation between a D-Lys amount in the horny layer and an L* value of the skin in the women in their twenties or thirties.
FIG. 4 is a diagram of a correlation between a D-Tyr amount in the horny layer and the L* value of the skin in the women in their forties or fifties.
FIG. 5 is a graph of an increase (a relative value to an increase of a control) of a melanin production-related protein gene (tyrosinase) of D-Asn (Example 2).
FIG. 6 is a graph of an increase (a relative value to an increase of a control) of a melanin production-related protein gene (tyrosinase-related protein 1) of D-Asn (Example 2).
FIG. 7 is graph of an increase (a relative value to an increase of a control) of a melanin production-related protein gene (melanocortin 1 receptor) of D-Asn (Example 1).
FIG. 8 is a graph of an increase (a relative value to an increase of a control) of the melanin production-related protein gene (tyrosinase) of D-Phe (Example 3).
FIG. 9 is a graph of an increase (a relative value to an increase of a control) of the melanin production-related protein gene (tyrosinase-related protein 1) of D-Phe (Example 3).
FIG. 10 is a graph of an increase (a relative value to an increase of a control) of the melanin production-related protein gene (tyrosinase-related protein 1) of D-Trp (Example 4).
FIG. 11 is a graph of cell survival rates in normal human epidermal melanocytes when D-Asn, D-Phe, and D-Trp are added.
FIG. 12 is a graph of black melanin production rates in B16 melanoma cells when D-Asp is added.
FIG. 13 is a graph of black melanin production rates in B16 melanoma cells when D-Glu is added.
FIG. 14 is a graph of black melanin production rates in B16 melanoma cells when D-Cys is added.
FIG. 15 is a graph of black melanin production rates in B16 melanoma cells when D-Trp is added.
FIG. 16 is a graph of cell survival rates in B16 melanoma cells when D-Asp is added.
FIG. 17 is a graph of cell survival rates in B16 melanoma cells when D-Glu is added.
FIG. 18 is a graph of cell survival rates in B16 melanoma cells when D-Cys is added.
FIG. 19 is a graph of cell survival rates in B16 melanoma cells when D-Trp is added.
FIG. 20 is a graph of a rate of a melanin production rate in normal human epidermal melanocytes when D-Cys is added.
FIG. 21 is a graph of a rate of a melanin production rate in normal human epidermal melanocytes when D-Asp is added.
FIG. 22 is a graph of rates of a melanin production rate in normal human epidermal melanocytes when D-His is added.
FIG. 23 is a graph of a rate of a melanin production rate in normal human epidermal melanocytes when D-Phe is added.
FIG. 24 is a graph of a rate of a melanin production rate in normal human epidermal melanocytes when D-Trp is added.
FIG. 25 is a graph of a relative ratio of a content of protein in normal human epidermal melanocytes when D-Cys is added.
FIG. 26 is a graph of a relative ratio of a content of protein in normal human epidermal melanocytes when D-Asp is added.
FIG. 27 is a graph of relative ratios of a content of protein in normal human epidermal melanocytes when D-His is added.
FIG. 28 is a graph of a relative ratio of a content of protein in normal human epidermal melanocytes when D-Phe is added.
FIG. 29 is a graph of a relative ratio of a content of protein in normal human epidermal melanocytes when D-Trp is added.

### Description of Embodiments

### [1. Whitening Agent]

In one embodiment, the present invention relates to the non-therapeutic use of a composition according to present claim 1 as a skin-whitening agent. The composition used as a skin-whitening agent in the invention contains at least one D-amino acid selected from the group consisting of D-Asn, D-Val, D-allo-Thr, D-Lys, D-Gln, D-His, D-Phe,**and** D-Trp, as an active ingredient. The active ingredient is preferably at least one D-amino acid selected from the group consisting of D-Asn, D-Val, D-allo-Thr, D-Lys, D-Gln, D-His,
D-Phe,**and** D-Trp, D-Cys may be low in stability like L-Cys (Japanese Patent Application Laid-open No. 2009-227660). For D-Asp and D-Glu, it may be required to adjust pH to be higher in order to improve solubility.

The inventors of the present invention have found out that there is a correlation between the content of the D-amino acid, which is contained only in an infinitesimal amount in living bodies, and a melanin index and an L* value of the skin and have studied the gene expression amount of proteins related to melanin production using the D-amino acid for which the correlation has been found to find a significant decrease in the gene expression amount of the proteins.

Consequently, a new knowledge has been found out that the D-amino acid, which is contained only in an infinitesimal amount in living bodies, among amino acids in which the amino acids as racemic bodies themselves have been conventionally considered to be active ingredients strongly inhibits the production of the proteins related to the melanin index and the L* value of the skin and acts as an active ingredient. The whitening agent of the present invention, based on such a new knowledge, uses a marked effect of the D-amino acid, which is contained only in an infinitesimal amount in living bodies.

In the present specification, "whitening" means inhibiting excessive occurrence of melanin of the skin to improve pigmented spot, freckles, and somberness.

The amino acid has a D-isomer and an L-isomer as optical isomers. Among these, natural proteins contain L-amino acids that are peptide-bonded. It is known that limonene has optical isomers that differ in physiological activity, for example. However, the influence of the D-amino acid on proteins containing L-amino acids is currently under study, and no systematic understanding has been established.

The D-amino acid may be a salt thereof. The salt may be any salt pharmaceutically allowed. Examples thereof include alkaline metal salts such as a potassium salt and a sodium salt; alkaline earth metal salts such as a calcium salt, a barium salt, and a magnesium salt; ammonium salts such as an ammonium salt and a tricyclohexylammonium salt; alkanol amine salts such as a monoethanolamine salt, a diethanolamine salt, a triethanolamine salt, a monoisopropyl alcohol amine salt, a diisopropyl alcohol amine salt, and a triisopropyl alcohol amine salt.

The method for producing the D-amino acid is not limited to a particular method. Examples thereof include a synthesis method, an extraction method, an enzyme method, and a fermentation method. Another example thereof is a method that optically resolves a racemic mixture of D,L-amino acids. The optical resolution is not limited to a particular method and may be performed by a conventionally known method (e.g., a method that prepares a diastereomer salt using an optically resolving agent such as camphorsulfonic acid and performs resolution using solubility difference).

The skin-whitening agent may contain any whitening ingredient other than the D-amino acids to form a composition of a whitening agent as needed.

The skin-whitening agent preferably contains at least either of at least one D-amino acid selected from the group consisting of D-Gln, D-His,
and D-Trp and a D-amino acid as D-Tyr and/or D-Lys as an active ingredient and more preferably contains at least either of at least one D-amino acid selected from the group consisting of D-Gln, D-His, and D-Trp and a D-amino acid as D-Tyr and/or D-Lys as an active ingredient. For these D-amino acids, a correlation with at least either a melanin index or an L* value of women of a young age has been recognized in a method of evaluation of the present invention described below.

The skin-whitening agent also
preferably contains
D-Phe, as an active ingredient or containing at least either of D-His and at least one D-amino acid selected from the group consisting of D-Gln, D-Trp, D-Asn, D-Val, D-allo-Thr, and D-Lys as an active ingredient. For these D-amino acids, a correlation with at least either a melanin index or an L* value of elderly women has been recognized in the method of evaluation of the present invention described below.

The efficacy of the skin-whitening agent
can be determined by a method described in quasi-drug whitening function evaluation test guidelines for the acquisition of novel efficacy described in Journal of Japanese Cosmetic Science Society, vol. 30, No. 4, pp. 333-337 (2006), for example. More specifically, it can be determined by a method that, in a pigmentation evaluation visually or through a photograph, determines inhibition of pigmentation or a method that determines decreasing a melanin index or increasing an L* value calculated from a spectroscopic colorimeter measured value. Among them, preferred is the method that decreases a melanin index or increases an L* value calculated from a spectroscopic colorimeter measured value.

### [1-1. Production Inhibitor of Melanin Production-Related Protein]

In certain embodiments the skin-whitening agent is a production inhibitor of at least one melanin production-related protein. The production inhibitor of a melanin production-related protein contains at least one D-amino acid selected from the group consisting of D-Asn, D-Phe, D-Trp, D-Glu, and D-His as an active ingredient. The active ingredient is preferably at least one D-amino acid selected from the group consisting of D-Asn, D-Phe, D-Trp, and D-His. The melanin production-related protein is at least one selected from the group consisting of tyrosinase, tyrosinase-related protein 1, and melanocortin 1 receptor. Consequently, the production of at least one melanin production-related protein selected from the group consisting of tyrosinase, tyrosinase-related protein 1, and melanocortin 1 receptor is inhibited, and thus pigmented spot, freckles, and somberness can be improved or inhibited. The D-amino acids may be salts thereof, which are as described above for the whitening agent.

The protein the production of which is inhibited by the production inhibitor of a melanin production-related protein is at least one selected from the group consisting of tyrosinase, tyrosinase-related protein 1, and melanocortin 1 receptor.

Tyrosinase is a protein strongly related to pigmented spot and catalyzes an initial reaction of melanin production.

Tyrosinase-related protein 1 is a protein strongly related to pigmented spot and catalyzes the step next to tyrosinase in melanin production.

Melanocortin 1 receptor is a protein strongly related to pigmented spot and is an α-melanocyte stimulating hormone (α-MSH) receptor.

The production inhibitor of a melanin production-related protein -may contain any production inhibitor of a melanin production-related protein other than D-Asn, D-Phe, D-Trp, D-Glu, and D-His to form a production inhibition composition of a melanin production-related protein as needed.

### [2. Skin Cosmetic and Skin External Preparation (Skin Cosmetic Composition and Skin External Preparation Composition)]

The compositions used in claims 1-5 may be skin cosmetic compositions or skin external preparation compositions.

The skin cosmetic and skin external preparation
contains the whitening agent or the production inhibitor of a melanin production-related protein. The content of the whitening agent or the production inhibitor of a melanin production-related protein may be adjusted as appropriate in accordance with the type of the skin cosmetic and skin external preparation. A preferred lower limit of the content in terms of the total amount of the D-amino acid is normally 0.01% by mass or more, preferably 0.1% by mass or more, and more preferably 1% by mass or more. The upper limit thereof is normally 30% by mass or less and preferably 10% by mass or less.

The skin cosmetic and skin external preparation
may further contain at least one base selected from the group consisting of a polyol compound, a surfactant, a higher alcohol, a thickener, an ultraviolet absorbing agent, an ultraviolet scattering agent, a chelating agent, an preservative, and a silicone and preferably contains it.

### (Polyol Compound)

Examples of the polyol compound include ethylene glycol, butylene glycol, polyethylene glycol, butyl ethyl propanediol, a polypropylene glycol copolymer, propylene glycol, dipropylene glycol, 1,3-butylene glycol, glycerin, diglycerin, and sorbitol. Among them, preferred is at least one selected from the group consisting of butylene glycol, glycerin, polyethylene glycol, dipropylene glycol, and sorbitol.

### (Surfactant)

Examples of the surfactant include anionic surfactants such as polyoxyethylene sorbitan oleate, higher fatty acid soaps, alkyl sulfates, polyoxyethylene alkyl ether sulfates, alkyl ether phosphates, N-acylamino acid salts, and acyl N-methyl taurine salts; cationic surfactants such as alkyltrimethylammonium chloride and dialkyldimethylammonium chloride; amphoteric surfactants such as cocamidopropyl betaine, alkyldimethylaminoacetic acid betaine, alkylamidodimethylaminoacetic acid betaine, and 2-alkyl-N-carboxy-N-hydroxyimidazolinium betaine; and nonionic surfactants such as glycerin fatty acid esters, polyglycerin fatty acid esters, lecithin derivatives (lysolecithin), polyoxyethylene type ones (polyoxyethylene hydrogenated castor oil, polyoxyethylene alkyl ethers, polyoxyethylene polyoxypropylene alkyl ethers, and polyoxyethylene glycerin fatty acid esters), polyhydric alcohol ester type ones, and an ethylene oxide/propylene oxide block copolymer. Among them, preferred is at least one selected from the group consisting of glycerin fatty acid esters, polyglycerin fatty acid esters, lecithin derivatives, polyoxyethylene hydrogenated castor oil, polyoxyethylene alkyl ethers, polyoxyethylene polyoxypropylene alkyl ethers, and polyoxyethylene glycerin fatty acid esters.

### (Higher Alcohol)

Examples of the higher alcohol include cetanol, behenyl alcohol, and isohexadecyl alcohol. Among them, behenyl alcohol is preferred.

### (Thickener)

Examples of the thickener include polyacrylic acid, carrageenan, xanthan gum, sodium carboxymethyl cellulose, carboxyvinyl polymer, polyoxyethylene glycol distearate, an acrylic acid-based polymer, a cellulose-based natural polymer, ethanol, neopentyl glycol dicaprate, and potassium myristate. Among them, preferred is at least one selected from the group consisting of an acrylic acid-based polymer, xanthan gum, and a cellulose-based natural polymer.

### (Ultraviolet Absorbing Agent)

Examples of the ultraviolet absorbing agent include butyl methoxybenzoylmethane, octyl p-dimethylaminobenzoate, cinnamic acid derivatives such as 2-ethylhexyl p-methoxycinnamate, and ethylhexyl dimethoxybenzylidene dioxoimidazolidine propionate. Among them, cinnamic acid derivatives are preferred.

### (Ultraviolet Scattering Agent)

For the ultraviolet scattering agent, at least either titanium oxide or zinc oxide is preferred.

### (Chelating Agent)

Examples of the chelating agent include etidronic acid and ethylenediaminetetraacetic acid (or derivatives thereof) such as trisodium edetate. Among them, ethylenediaminetetraacetic acid is preferred.

### (Preservative)

For the preservative, at least either paraben or phenoxy ethanol is preferred. Examples of paraben include methylparaben, propylparaben, and butylparaben.

### (Silicone)

Examples of the silicone include methylpolysiloxane, dimethylpolysiloxane, methylphenylpolysiloxane, decamethylcyclopentasiloxane, cyclohexasiloxane, cyclopentasiloxane, trimethylsiloxysilicate, a polyoxyethylene/methylpolysiloxane copolymer, silicone fluid, silicone rubber, and silicone oil.

The skin cosmetic or skin external preparation
may contain one or two or more bases other than the above. The other bases may be bases used for normal skin cosmetics or skin external preparations; examples thereof include oil-based bases, water-soluble bases, emulsifiers, stabilizers, pH regulators, preservatives, other functional components (physiologically active components), perfumes such as triethylhexanoin, pigments, solvents, natural product extracts, and amino acids (derivatives) other than the above D-amino acids.

Examples of oil-based bases include vegetable oils such as castor oil, cottonseed oil, sesame oil, jojoba oil, olive oil, and cacao butter; waxes such as Japan wax, lanolins such as liquid lanolin, candelilla wax, beeswax, and carnauba wax; higher hydrocarbons such as paraffins such as Vaseline, liquid paraffin, and solid paraffin, squalane, olefin oligomers, and plastibase; fatty acids such as lauryl acid, myristic acid, stearic acid, and palmitic acid and esters thereof; base waxes such as kerosene; and natural polymers and hydrogenated polyisobutene.

Examples of water-soluble bases include polyvinyl alcohol, cellulose derivatives (e.g., methylcellulose, hydroxyethyl cellulose, hydroxypropyl cellulose, and cationized cellulose), carboxyvinyl polymer, and esters (e.g., isopropyl myristate, isopropyl palmitate, stearyl stearate, octyldodecyl myristate, octyldodecyl oleate, and triglyceride 2-ethylhexanoate).

Examples of emulsifiers include stearyl alcohol, glyceryl monostearate, sorbitan monopalmitate, polyoxyethylene cetyl ether, polyoxyethylene sorbitan monolaurate, polyoxyethylene sorbitan monooleate, diglycerin monostearate, polysorbate 60, and phytosteryl/decyltetradecyl myristoyl methyl-β-alanine.

Examples of stabilizers include spherical alkyl polyacrylate powder, dimethyldistearylammonium hectorite, ascorbic acid, sodium pyrosulfite, gellan gum, and pectin.

Examples of pH regulators include a phosphate buffer, sodium hydroxide, potassium hydroxide, and triethanolamine.

Examples of preservatives include ethyl paraoxybenzoate, sodium benzoate, salicylic acid, sorbic acid, sodium hydrogen sulfite, and phenoxy ethanol.

Examples of solvents include an aqueous arginine solution and purified water.

Examples of pigments include Red 202, Yellow 4, Blue 1, Bengala, yellow iron oxide, and black iron oxide.

Examples of other functional components include kojic acid, trioleyl phosphate, squalane, cetyl ethylhexanoate, tocopherol, mica, dimer dilinoleyl bis(N-lauroyl-L-glutamate/N-lauroyl sarcosinate), tocopherol acetate, thiotaurine, honey, lauroylsarcosine isopropyl, lauroyl lysine, talc, dibutyl ethylhexanoyl glutamide, dibutyl lauroyl glutamide, bentonite, taurine, arbutin, panthenol, niacinamide, pyridoxine hydrochloride, retinol, carotene, riboflavin, ubiquinone, sodium hyaluronate, water-soluble collagen, sodium chondroitin sulfate, lactobacillus, milk ferment filtrate, whey, yogurt extract, sodium hydrolyzed casein, soybean ferment extract, benzalkonium chloride, triclosan, salicylic acid, citric acid, bisabolol, calcium chloride, glyceryl caprylate, mineral oil, sodium lauroyl glutamate, cocamide MEA, cocamide methyl MEA, polyvinylpyrrolidone, sodium cocoamphoacetate, polyquaternium, and guar hydroxypropyltrimonium chloride.

Examples of natural product extracts include lavender extract, peppermint extract, sage extract, anise extract, rose extract, Chamaecyparis obtusa water, rooibos extract, lavender extract, and Sophora angustifolia root extract.

Examples of amino acids (derivatives) include L-leucine, L-isoleucine, L-valine, L-phenylalanine, L-tryptophan, L-aspartic acid, sodium L-glutamate, L-lysine (hydrochloride), L-arginine, L-threonine, L-alanine, L-proline, L-serine, glycine, acetylglutamine, L-histidine, pyridoxylserine, carnosine, acetylmethionine, acetylcysteine, sodium polyaspartate, citrulline, ornithine, betaine, L-tyrosine, L-asparagine, and L-methionine.

The content of the other bases, which may be set as appropriate, is not limited to a particular content.

The dosage form of the skin cosmetic or skin external preparation is not limited to a particular form; examples thereof include liquid, lotion, ointment, cream, plaster, tape formulation, powder, skin lotion, and gel. Among them, examples thereof include milky lotion, cream, skin lotion, and gel, and any of these is preferred.

The use site of the skin cosmetic or skin external preparation is not limited to a particular site; it is normally applied to the skin and the mucosae. Among them, it is preferably used for the skin. Given this, examples of the use form of the skin cosmetic or skin external preparation (
include aqueous solution, emulsion, powder dispersion, and emulsion (oil-in-water type, water-in-oil type, and the like). More specifically, examples thereof include liquid, oil, lotion, gel, sol, milky lotion, suspension, cream, ointment, patch, and stick. Examples of what is called cosmetics include skin lotions such as lotions and beauty liquids; milky lotions such as emollient milky lotions, milky lotions, nourishing milky lotions, and cleansing milky lotions; creams such as emollient creams, massage creams, cleansing creams, and makeup creams; sprays; cosmetics for makeup such as packs, foundations, rouges, lipsticks, eye shadows, blushers, white powders, and color powders and cosmetics for skin washing such as face cleansing agents, makeup removers, body shampoos, and soaps. The skin cosmetic or skin external preparation
may be used as quasi-drugs, drugs, and foods (including supplements and drinks) apart from the cosmetics.

Examples of the formulation of the skin cosmetic or skin external preparation as a milky lotion or a cream include a formulation containing the agent of the present invention, a polyol group (at least one selected from the group consisting of butylene glycol, glycerin, polyethylene glycol, and dipropylene glycol), a surfactant group (at least one selected from the group consisting of glycerin fatty acid esters, polyglycerin fatty acid esters, and lecithin derivatives), a higher alcohol group (behenyl alcohol), a thickener group (at least either an acrylic acid-based polymer or xanthan gum), an ultraviolet absorbing agent group (cinnamic acid derivatives), an ultraviolet scattering agent group (at least either titanium oxide or zinc oxide), a chelating agent group (ethylenediaminetetraacetic acid), an preservative group (at least either paraben or phenoxy ethanol), and a silicone.

Examples of the formulation of the skin cosmetic or skin external preparation as a skin lotion include a formulation containing the agent of the present invention, a polyol group (at least one selected from the group consisting of butylene glycol, glycerin, polyethylene glycol, and sorbitol), a surfactant group (at least one selected from the group consisting of polyoxyethylene hydrogenated castor oil, polyoxyethylene alkyl ethers, polyoxyethylene polyoxypropylene alkyl ethers, and polyoxyethylene glycerin fatty acid esters), a thickener group (at least one selected from the group consisting of an acrylic acid-based polymer, xanthan gum, and a cellulose-based natural polymer), an ultraviolet absorbing agent group (cinnamic acid derivatives), a chelating agent group (ethylenediaminetetraacetic acid), an preservative group (at least either paraben or phenoxy ethanol), and a silicone.

Examples of the formulation of the skin cosmetic or skin external preparation as a gel include a formulation containing the agent of the present invention, a polyol group (at least one selected from the group consisting of butylene glycol, glycerin, and polyethylene glycol), a surfactant group (at least one selected from the group consisting of polyoxyethylene hydrogenated castor oil, polyoxyethylene alkyl ethers, polyoxyethylene polyoxypropylene alkyl ethers, and polyoxyethylene glycerin fatty acid esters), a thickener group (at least one selected from the group consisting of an acrylic acid-based polymer, xanthan gum, and a cellulose-based natural polymer), an ultraviolet absorbing agent group (cinnamic acid derivatives), a chelating agent group (ethylenediaminetetraacetic acid), an preservative group (at least either paraben or phenoxy ethanol), and a silicone.

The whitening agent and the production inhibitor of a melanin production-related protein of the present invention can exhibit an excellent effect for prevention, inhibition, and treatment of pigmented spot, freckles, and somberness caused by aging, ultraviolet rays, inflammation, and the like through a whitening action.

In one embodiment the present invention relates to the non-therapeutic use of a composition according to claim 5 in a method or prevention, inhibition, or treatment of pigmented spot, freckles, or somberness.

### [3. Method of Evaluation]

A method of evaluation of the present invention is a method of evaluation of a skin by a D-amino acid including measuring at least either a melanin index or an L* value of a skin of a subject to determine an appearance of the skin of the subject, collecting a horny layer from a pigmentation site of the skin of the subject, identifying at least one D-amino acid level contained in the horny layer, and comparing a relation between the appearance of the skin and the D-amino acid level among results of the subject.

The method of evaluation of the present invention broadly includes the following two embodiments. One embodiment is an embodiment that compares a relation between the appearance of the skin and each component among results of a subject (a spare subject) (which may be a comparison among a plurality of results of the same subject or a comparison among results of a plurality of different subjects), determines whether there is a correlation, and, when a correlation is determined, selects a correlation between the appearance of the skin and the D-amino acid level as an indicator of the evaluation of the skin of the subject (which may be the same as or different from the spare subject). The other embodiment is an embodiment that identifies a D-amino acid level in keratin before and after a subject is dosed with a sample of a skin cosmetic, a skin external preparation, a candidate substance thereof, or the like and compares evaluations of the skin before and after dosing obtained in light of the above correlation with each other to perform an evaluation of the sample or an evaluation of efficacy to the skin of the subject.

The subject may be a man or a woman; however, the subject is preferably a woman because of sensitiveness to whitening and is more preferably a woman in her twenties or thirties who begins to have interest in pigmented spot or somberness by aging or a woman in her forties or fifties who often take care of pigmented spot or somberness by aging.

In the method of evaluation of the present invention, the appearance of the skin may be determined by at least either the melanin index or the L* value.

In persons having pigmented spot or somberness, owing to a reduction in the function of skin cells, in particular, a reduction in a cell function (a cell differentiation and proliferation function) caused by a melanin production increase in melanocytes caused by external stimuli such as exposure to ultraviolet rays and subsequent excessive transport and accumulation of melanin to and in keratinocytes, a melanin amount in the keratinocytes is apt to increase. For this reason, the melanin index is used as an indicator evaluating the absence of pigmented spot and somberness, that is, the whitening of the skin.

The L* value is an indicator representing color brightness and is used as an indicator evaluated with a value of 0 to 100; 0 indicates a black color, whereas 100 indicates a white, diffusion color, and it can be said that a larger value indicates a more excellent whitening effect.

Measurement of the melanin index and the L* value may be performed by a conventionally known method.

Examples thereof include measurement using a combination of a spectroscopic colorimeter CM-700d (manufactured by Konica Minolta, Inc.) that can measure reflected light in a range of 400 to 700 nm using a pulse xenon lamp as a light source and skin analysis software CM-SA for data analysis. It can be said that a smaller melanin index indicates a more excellent whitening effect. The appearance of the skin may be evaluated by the density of melanin that can be measured from light reflected when the skin is irradiated with light with a wavelength of 660 nm or 880 nm using MexameterMX18 of Courage+Khazaka.

The measurement site of the appearance of the skin is not limited to a particular site; however, it is preferably a site exposed to the outside in daily lives in view of evaluating pigmented spot, freckles, and somberness caused by excessive melanin production caused by external stimuli such as ultraviolet rays. Examples thereof include a cheek and an arm.

The measurement site may be one or two or more.

The horny layer may be collected from a pigmentation site of the appearance of the skin by a conventionally known method such as tape stripping. Tape stripping is a method that puts adhesive tape on the skin and peels off the adhesive tape to collect a horny layer attached to an adhesive face.

Examples of the method for collecting the horny layer include a method that, when commercially available adhesive tape is pressed against a measurement site and is then removed therefrom, collects a horny layer attached to the adhesive layer. Examples of the commercially available adhesive tape include Horny Layer Checker manufactured by Asahi Biomed Co., Ltd., Horny Layer Seal manufactured by Moritex Corporation, Horny Layer Checker (Disc Type W, Disc Type G, and PRO Type) manufactured by Promotool Corporation, Corneofix manufactured by Integral Corporation, Transparent Tape manufactured by 3M, Transparent Double-Sided Tape manufactured by 3M, and Cellotape (registered trademark) manufactured by Nichiban Co., Ltd.

The method for identifying the D-amino acid level is not limited to a particular method; examples thereof include a direct method (a method that performs precolumn derivatization and then performs separation using chromatography with a chiral stationary phase column) or an indirect method (a method that performs derivatization to a diastereomer with a chiral derivatizing reagent and then performs separation using chromatography with a reversed phase column).

Examples of the direct method include a method that performs precolumn fluorescence derivatization with NBD-F, then performs separation with a chiral column, and performs fluorescence detection. The details thereof can be referred to "Comprehensive analysis of branched aliphatic D-amino acids in mammals using an integrated multi-loop two-dimensional column-switching high-performance liquid chromatographic system combining reversed-phase and enantioselective columns." J Chromatogr A. 2007 Mar 2; 1143(1-2): 105-11. Hamase K, Morikawa A, Ohgusu T, Lindner W, Zaitsu K.

Examples of the indirect method include a method that performs derivatization with OPA and chiral thiol, performs separation with a reversed phase column, and performs fluorescence detection and a method that performs derivatization with DBD-PyNCS, performs separation with a reversed phase column, and performs detection with a mass spectrometer. The details thereof can be referred to "High-performance liquid chromatography analysis of naturally occurring D-amino acids in sake." J Chromatogr B Analyt Technol Biomed Life Sci. 2011 Nov 1;879(29):3259-67. Gogami Y, Okada K, Oikawa T. "Determination of DL-amino acids, derivatized with R(-)-4-(3-isothiocyanatopyrrolidin-1-yl)-7-(N,N-dimethylaminosulfonyl)-2,1,3-benz oxadiazole, in nail of diabetic patients by UPLC-ESI-TOF-MS" J. Chromatogr. B, 879, 3220-3228 (2011). J. Z. Min, S. Hatanaka, H.F. Yu, T. Higashi, S. Inagaki, T. Toyo'oka.

The D-amino acid to be identified preferably contains at least one selected from the group consisting of D-Asn, D-Val, D-allo-Thr, D-Lys, D-Gln, D-His, D-Leu, D-Phe, D-Pro, D-Ser, D-Trp, and D-Tyr.

It can be determined from the method of evaluation of the present invention that the more preferred D-amino acids are components having a correlation with at least either the melanin index or the L* value of the skin of women of at least either of twenties to thirties and forties to fifties.

The one embodiment of the method of evaluation of a skin by a D-amino acid of the present invention provides a new knowledge about a correlation between a specific D-amino acid and the appearance of the skin. Consequently, the method of evaluation of the present invention can be applied to development of skin cosmetics or skin external preparations such as whitening agents derived from natural products.

More specifically, the following can be said about the age of the subject and the type of the D-amino acid. When the subject is a woman in her twenties or thirties, the component of the D-amino acid is preferably at least one selected from the group consisting of D-Lys, D-Gln, D-His, D-Pro, D-Trp, and D-Tyr.

When the subject is a woman in her forties or fifties, the D-amino acid is preferably at least one selected from the group consisting of D-Asn, D-Val, D-allo-Thr, D-Lys, D-Gln, D-His, D-Leu, D-Phe, D-Ser, D-Trp, and D-Tyr.

The method for identifying a D-amino acid level is not limited to a particular method. Examples thereof include mass spectroscopy using liquid chromatography (LC) or gas chromatography (GC), an enzyme method using an enzyme for quantification, and bioassay method.

Among those described above, a liquid chromatographtandem mass spectrometer is preferred, because identification of the D-amino acid and quantification of the content thereof can be simultaneously performed.

The D-amino acid level is preferably a content of the D-amino acid per content of protein contained in the horny layer. With this D-amino acid level, experimental errors derived from the method for collecting the horny layer can be reduced.

The method for measuring the content of protein is not limited to a particular method; examples thereof include methods of measurement using a spectrophotometer such as an ultraviolet absorption method, the BCA method, the Bradford method, the Lowry method, and the Biuret method and a method of measurement by electrophoresis. Among them, the BCA method is preferred.

The method of evaluation of an appearance of a skin by a D-amino acid of the present invention obtains a relation between at least either the melanin index or the L* value of the skin and the D-amino acid level from a plurality of subjects, compares them with each other, and can thereby identify a D-amino acid that correlates with the appearance of the skin.

By using the method of evaluation of an appearance of a skin by a D-amino acid of the present invention, the appearance of the skin can be evaluated by the content of the D-amino acid. Consequently, before and after the same subject is dosed with a sample of a skin cosmetic or skin external preparation or the like, the contents of the D-amino acid for which the correlation has been recognized are compared with each other, whereby the efficacy of the sample to the subject can be determined. Consequently, the method of evaluation of the present invention can also be used as a method that determines whether the skin cosmetic or skin external preparation is appropriate for the subject.

The other embodiment of the method of evaluation of an appearance of a skin by a D-amino acid of the present invention can provide not only a short-term evaluation by the reduction in the melanin index or the increase in the L* value like conventional methods of measurement but also an evaluation about whether there is a possibility of a whitening effect by predicting a decrease in the melanin index or an increase in the L* value in the long term by the content of the D-amino acid even if no decrease in the melanin index or no increase in the L* value is observed immediately after dosing before and after being dosed with the skin cosmetic or skin external preparation.

### [Examples]

The following describes the present invention in detail with reference to Examples; the Examples do not limit the present invention.

### (Measurement of Melanin Index and L* Value)

Subjects washed their faces and were adapted in a constant-temperature constant-humidity room (humidity: 40%, temperature: 20°C) for 20 minutes.

After adaptation, the cheek central part of the subjects was measured using a spectroscopic colorimeter CM-700d (manufactured by Konica Minolta, Inc.) and skin analysis software CM-SA for data analysis.

### (Horny Layer Collection by Tape Stripping)

After the end of the measurements, a piece of tape cut into 3 cm × 3 cm was attached to the cheek central part of the subjects to collect a horny layer. A horny layer on the outermost layer collected for the first time was not used for analysis, and a horny layer collected for the second time or later was used for analysis.

### (D-Amino Acid Extraction Operation)

A piece of tape (3 × 3 cm²) was attached to the same site of the cheek, where the skin conditions (the melanin index and the L* value) were measured, of the subjects (all were women, age structure: twenties to thirties 31, forties to sixties 40; a total number of 71), and a horny layer was collected by tape stripping. The collected first layer horny layer was not used for analysis, and the second or third layer was used for analysis. The collected tape was put into a conical tube (50 ml), was hermetically sealed, and was stored at -80°C.

The collected horny layer was subjected to ultrasonic treatment twice using a 95% aqueous methyl alcohol solution, and a water-soluble component was extracted. To the extracted liquid, an internal standard liquid (containing a plurality of D,L-amino acid stable isotopes such as D-Arg) was added. The obtained aqueous methyl alcohol solution was dried and solidified with a centrifugal evaporator to be a sample for D-amino acid analysis.

### (Quantitative Analysis of D-Amino Acid)

Water in an amount of 50 µL was added to the concentrated and dried and solidified sample, which was stirred with a vortex mixer to be redissolved to be a sample solution. A borate buffer solution with a concentration of 200 mM (pH 8.8) in an amount of 10 µL was added to 20 µL of the sample solution, which was stirred, and then 20 µL of a 5 mg/mL (R)-N-(hydroxysuccinimidyl carbamate)-**β**-(3-pyridyl)-alanine methyl ester/acetonitrile solution was added thereto, and the solution was reacted at room temperature for 10 minutes. A 0.1 M aqueous hydrochloric acid solution in an amount of 60 **µ**L was added to the reaction solution to be a sample for analysis. The analysis was performed using LC/MS/MS under the following conditions.

### (HPLC Conditions)

Apparatus: Nexera X2 (manufactured by Shimadzu Corporation)
Guard column: Waters ACQUITY BEH C18 VanGuard (2.1 × 50 mm, 1.7 µm)
Column: Waters ACQUITY UPLC BEH C18 (2.1 × 100 mm, 1.7 µm)
Mobile Phase A: 10 mM aqueous ammonium bicarbonate solution (pH 9.5)
Mobile Phase B: 80% methanol/water
Flow rate: 0.5 mL/min
Column temperature: 50°C
Sample injection amount: 1 µL

Table 1 shows a gradient condition.

**[Table 1]**

| Time (min) | B (%) |
|---|---|
| 0.1 | 2 |
| 4 | 8 |
| 6 | 13.5 |
| 11 | 30 |
| 14 | 32 |
| 16 | 37 |
| 17 | 80 |
| 18 | 80 |
| 18.1 | 2 |
| 20 | 2 |

### (Mass Analysis Conditions)

Apparatus: Triple Quad 6500 (SCIEX)
Method of Ionization: ESI, Positive mode
Scan type: SRM (Selected Reaction Monitoring)

Table 2 shows a transition.

**[Table 2]**

| amino acids | Q1 | Q3 |
|---|---|---|
| D,L-Ala | 296.0 | 207.0 |
| D,L-Ser | 312.0 | 207.0 |
| D,L-Asp | 340.1 | 207.0 |
| D,L-Asn | 339.1 | 207.1 |
| D,L-Trp | 411.2 | 207.1 |
| D,L-Gln | 353.1 | 207.2 |
| D,L-Phe | 372.2 | 207.1 |
| D,L-Glu | 354.1 | 207.2 |
| D,L-Pro | 322.1 | 207.0 |
| D,L-Arg | 381.2 | 201.1 |
| D,L-Val | 324.1 | 207.1 |
| D,L-Leu | 338.1 | 207.1 |
| D-allo-Ile,,L-Ile | 338.1 | 207.1 |
| D-allo-Thr,,L-Thr | 326.1 | 207.0 |
| D,L-His | 362.1 | 181.1 |
| D,L-Lys | 559.2 | 181.0 |
| D,L-Met | 356.1 | 207.2 |
| D,L-Tyr | 594.2 | 388.2 |
| Gly | 282.1 | 207.0 |

### (Protein Extraction)

The tape after the water-soluble component was extracted was put into a microtube, and an insoluble fraction was eluted using hexane. After the tape was removed, the insoluble fraction was washed with hexane to be a sample for protein quantitative analysis.

### (Protein Quantitative Analysis)

An aqueous NaOH (0.5 mol/L)-SLS (0.05 wt%) solution was added to a tube used in "D-Amino Acid Extraction Operation" or the like and was heated for 60 minutes at 90°C. A protein eluted in these solutions was quantified.

For the quantification of the protein, Micro BCA Protein Assay Reagent (Thermo Fisher Scientific) was used, and the Micro BCA method was performed. The same amount of a sample solution and a Micro BCA solution were added and were shaken (1,100 rpm) while being heated at 50°C for 1 hour. Absorbance (562 nm) was measured with a spectrometer to quantify the protein.

### Examples and Comparative Examples of Method of Evaluation

Table 3 lists correlations between the contents of D-Asn, D-Val, D-allo-Thr, D-Lys, D-Gln, D-His, D-Leu, D-Phe, D-Pro, D-Ser, D-Trp, and D-Tyr in the horny layer and the melanin index and the L* value of the skin. Cases with an absolute value of a correlation coefficient of 0.3 or more were evaluated to be "A" as having "a medium correlation", whereas cases with that of 0.2 or more were evaluated to be "B" as having "a weak correlation."

**[Table 3]**

| | Blotch | | | |
|---|---|---|---|---|
| | Melanin index | | L* value | |
| | Young woman | Elderly woman | Young woman | Elderly woman |
| D-Gln | B | | | B |
| D-His | B | B (FIG 2) | | |
| D-Leu | | B | | B |
| D-Phe | | B | | A |
| D-Pro | B (FIG 1) | | | |
| D-Ser | | B | | B |
| D-Trp | B | | | A |
| D-Tyr | | A | B | B (FIG 4) |
| D-Asn | | | | A |
| D-Val | | | | B |
| D-allo-Thr | | | | B |
| D-Lys | | | B(FIG 3) | B |

As can be seen from Table 3, the contents of the D-amino acids, that is, D-Gln, D-His, D-Leu, D-Phe, D-Pro, D-Ser, D-Trp, and D-Tyr, in the horny layer were recognized to have correlations with at least either young women or elderly women in the melanin index of the skin. The contents of the D-amino acids, that is, D-Gln, D-Leu, D-Phe, D-Ser, D-Trp, D-Tyr, D-Asn, D-Val, D-allo-Thr, and D-Lys, in the horny layer were recognized to have correlations with at least either young women or elderly women in the L* value of the skin.

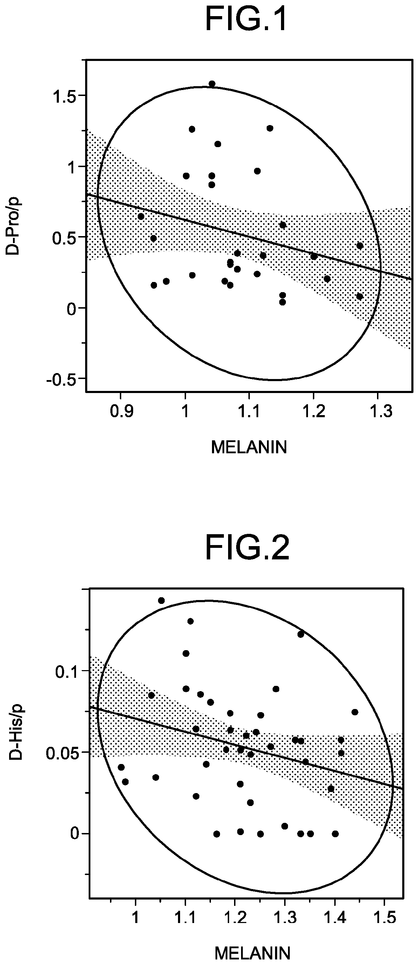

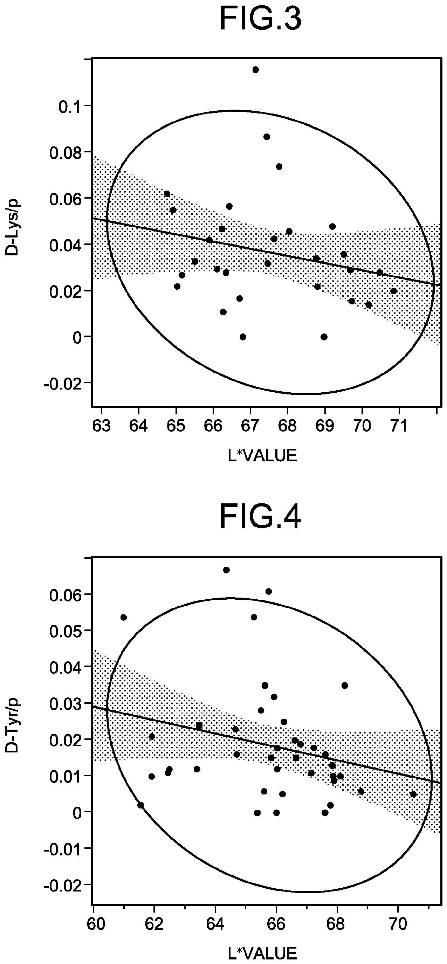

FIGS. 1 and 2 illustrate plots comparing the melanin index and the content of the D-amino acid with each other, whereas FIGS. 3 and 4 illustrate plots comparing the L* value and the content of the D-amino acid with each other. In FIGS. 1 to 4, the content of the amino acid is a value obtained by dividing the content of the amino acid determined in "Quantitative Analysis of D-Amino Acid" by the content of protein determined in "Protein Quantitative Analysis."

### Examples 1 to 4 of Whitening Agent

### (Gene Expression Test on "Whitening" of D-Amino Acid Using Normal Human Epidermal Melanocytes)

Normal human epidermal melanocytes (Life Technologies) were cultured with Medium 254 (Life Technologies) (containing HMGS custom-made multiplication additive set (Kurabo Industries Ltd.)) at 37°C and 5% CO₂ under saturated vapor. Cells that have become confluent were seeded in a 6-well plate with a seeding density of 3.0 × 10⁵ (cells/well).

For an evaluation sample, each D-amino acid adjusted in the culture medium so as to be 500 µM was used. On the next day of seeding, each evaluation sample was adjusted to be 500 µM, and each well culture medium was exchanged. Culturing was performed for 24 hours, and RNA was extracted from the cells using RNeasy mini kit (Qiagen), which was synthesized into cDNA using High-Capacity cDNA Reverse Transcription Kit with RNase Inhibitor (Thermo Fisher). With this cDNA as a template, a sample added group and a control group were compared with each other by the RT-PCR method (7500 fast system (Thermo Fisher Scientific)) to evaluate increase and decrease in a gene expression amount. The gene expression amount of at least either tyrosinase or tyrosinase-related protein 1 was evaluated. Table 4 lists results. In Table 4, TYR, TRP1, MC1R indicate tyrosinase, tyrosinase-related protein 1, and melanocortin 1 receptor, respectively.

**[Table 4]**

| | | Concentration (µM) | TYR | TRP1 | MC1R |
|---|---|---|---|---|---|
| Example 1 | D-Asn | 100 | | | 0.83 |
| Example 2 | | 500 | 0.34 | 0.25 | |
| Example 3 | D-Phe | 500 | 0.75 | 0.70 | |
| Example 4 | D-Trp | | | 0.64 | |

From Table 4, when D-Asn was used, a reduction in the expression amount of a melanocortin 1 receptor gene in a concentration of 100 µM and reductions in the expression amounts of tyrosinase and tyrosinase-related protein 1 genes in a concentration of 500 µM were recognized. When D-Phe was used, reductions in the expression amounts of the tyrosinase and tyrosinase-related protein 1 genes in a concentration of 500 µM were recognized. When D-Trp was used, a reduction in the expression amount of the tyrosinase-related protein 1 gene in a concentration of 500 µM was recognized.

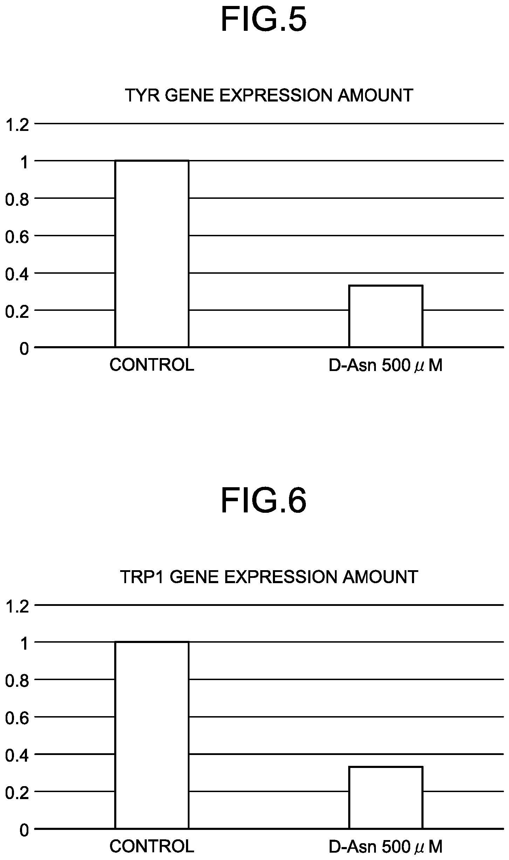

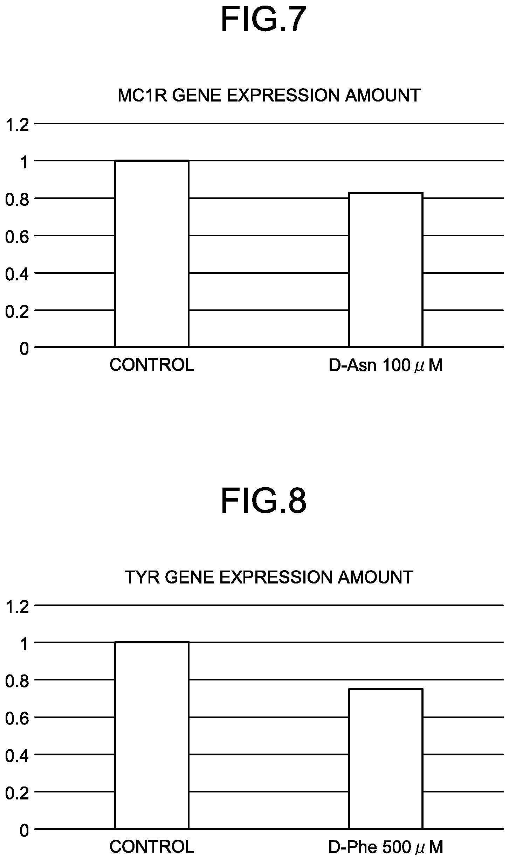

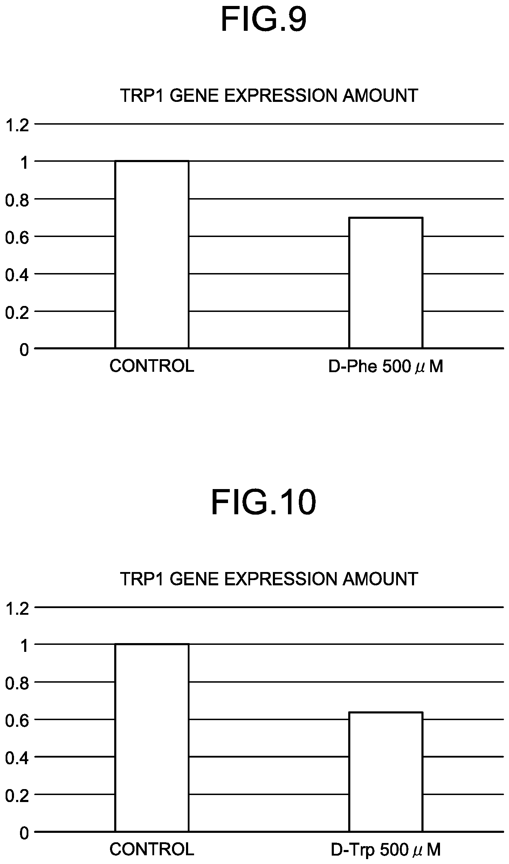

FIG. 5 is a graph of an increase (a relative value to an increase of a control) of a melanin production-related protein gene (tyrosinase) of D-Asn (Example 2). FIG. 6 is a graph of an increase (a relative value to an increase of a control) of a melanin production-related protein gene (tyrosinase-related protein 1) of D-Asn (Example 2). FIG. 7 is graph of an increase (a relative value to an increase of a control) of a melanin production-related protein gene (melanocortin 1 receptor) of D-Asn (Example 1). FIG. 8 is a graph of an increase (a relative value to an increase of a control) of a melanin production-related protein gene (tyrosinase) of D-Phe (Example 3). FIG. 9 is a graph of an increase (a relative value to an increase of a control) of a melanin production-related protein gene (tyrosinase-related protein 1) of D-Phe (Example 3). FIG. 10 is a graph of an increase (a relative value to an increase of a control) of a melanin production-related protein gene (tyrosinase-related protein 1) of D-Trp (Example 4).

These results show that the D-amino acid inhibits the production of the melanin production-related proteins and is useful for an active ingredient of the whitening agent.

### Example 5

### (Cytotoxicity Test: Neutral Red Method)

Normal human epidermal melanocytes were seeded in a 6-well plate, and on the next day, a sample with a D-amino acid (500 µM) adjusted in a cell culturing culture medium was added thereto. After 24 hours, the evaluation sample solution was removed from the plate, and each well was rinsed using 2 ml of a cell culture medium. A culture medium with Neutral Red (NR) in it (Kurabo Industries Ltd.) was added dropwise with 2 ml/well and was allowed to stand for 2 hours at 37°C and 5% CO₂ under saturated vapor.

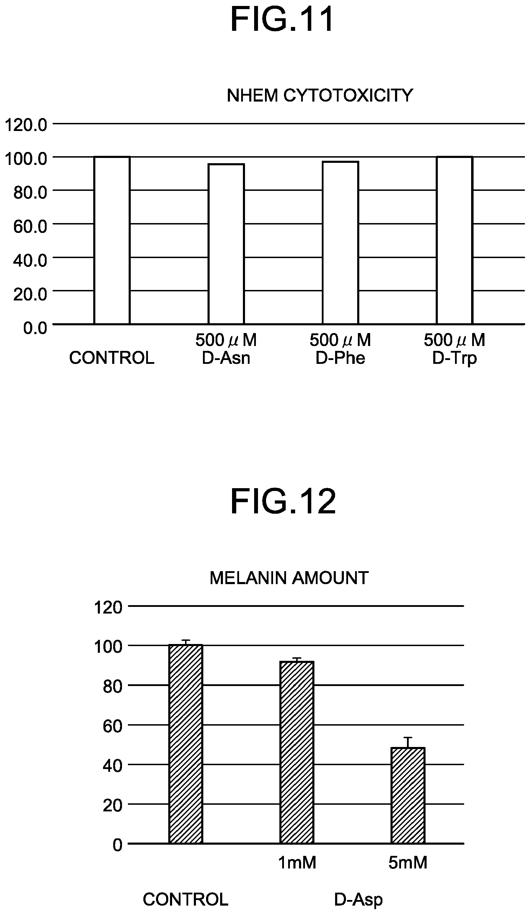

The culture medium (with Neutral Red in it) was discarded, a washing fixative (a 2 wt% calcium chloride solution and a 2 wt% formalin solution mixed with the equal amount) was added with 2 ml/well, and after being allowed to stand for 1 minute, the washing fixative was removed. An NR extract (50% by volume ethyl alcohol and 1% by volume acetic acid) was added with 2 ml/well and was shaken with a plate shaker for 15 minutes. Inclusion of NR into living cells was examined by measuring the absorbance at 540 nm of the NR extract with a microplate reader (manufactured by Thermo Fisher Scientific). The absorbance of the NR extract of each sample-added cell with a certain concentration when the measured value (absorbance) of the NR extract of a control (without sample addition) was defined as 100% was represented as relative percentage, whereby the cytotoxicity of each sample was calculated. FIG. 11 illustrates evaluation results.

It can be seen from FIG. 11 that each D-amino acid component is free of cytotoxicity in a concentration of 500 µM. Consequently, it has been found out that, in the concentration free of cytotoxicity, the D-amino acid can reduce the gene expression amount of various kinds of melanin production-related proteins.

### Example 6

### (Black Melanin Production Inhibition Test 1)

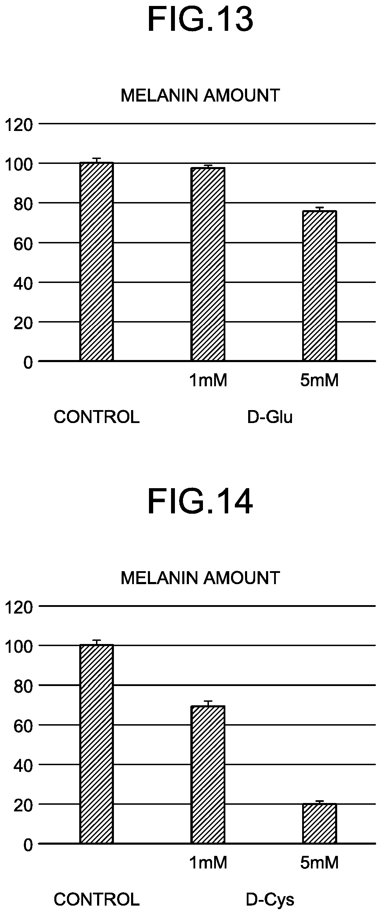

B16 melanoma cells were cultured with Dulbecco's Modified Eagle Medium (DMEM) (high glucose, containing 10% blood serum). Cells that have become confluent were peeled off with trypsin and were seeded in a 96-well plate. On the next day, after the cells adhered to the plate, culture medium exchange with DMEM (high glucose, containing 10% blood serum, and containing no Phenol Red) with each evaluation sample (a control (without sample addition) and the D-amino acid (any of D-Asp, D-Glu, D-Cys, and D-Trp)) added in certain evaluation concentrations (1 mM or 5 mM) was performed, which was cultured for 3 days. The 96-well plate was shaken with a plate shaker for 5 minutes, and absorbance at 450 nm was measured with a microplate reader (Multiskan FC manufactured by Thermo Fisher Scientific). The absorbance 3 days after addition of each sample with a certain concentration when the measured value (absorbance) of the control (without sample addition) was defined as 100% was represented as relative percentage, whereby a black melanin production rate of each sample with a black melanin amount in the control as 100% was calculated. FIGS. 12 to 15 illustrate results.

It can be seen from FIGS. 12 to 15 that, in an evaluation concentration of 1 mM or 5 mM of each sample, the addition thereof reduces the black melanin production rate of the cells.

### Example 7

### (Cytotoxicity Test (Neutral Red Method))

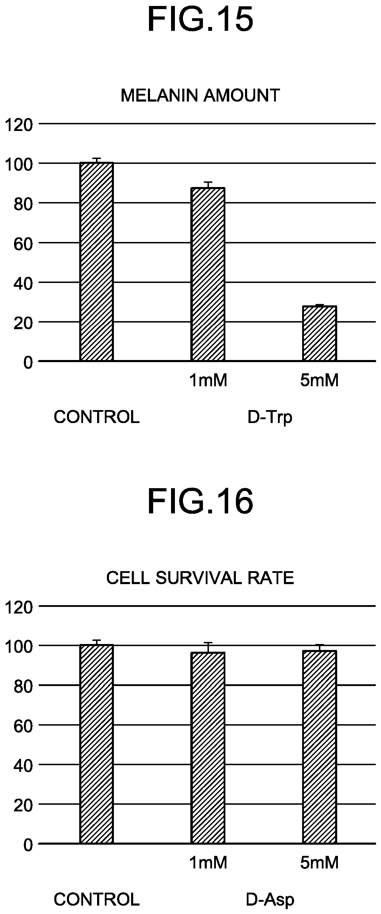

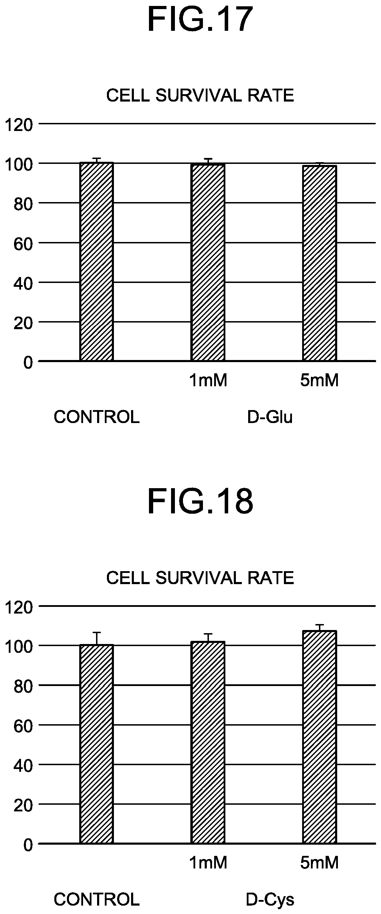

After absorbance was measured in Example 6, the evaluation sample solution was removed from the plate, and each well was rinsed using 200 µL of DMEM (high glucose, containing 10% blood serum). A culture medium with Neutral Red (NR) (0.5%) in it was added dropwise with 200 µl/well and was allowed to stand for 2 hours at 37°C and 5% CO₂ under saturated vapor. The culture medium was discarded, a washing fixative (a 2 wt% calcium chloride solution and a 2 wt% formalin solution mixed with the equal amount) was added with 200 µl/well, and after being allowed to stand for 1 minute, the washing fixative was removed. An NR extract (acetic acid acidic ethanol) was added with 200 µl/well and was shaken with a plate shaker for 15 minutes. Inclusion of NR into living cells was examined by measuring the absorbance at 540 nm of the NR extract with a microplate reader (Multiskan FC manufactured by Thermo Fisher Scientific). The absorbance of the NR extract of each sample-added cell with a certain concentration when the measured value (absorbance) of the NR extract of a control (without sample) was defined as 100% was represented as relative percentage, whereby a cell survival rate of each sample was calculated. FIGS. 16 to 19 illustrate results.

It can be seen from FIGS. 16 to 19 that the samples are all low in cytotoxicity in an evaluation concentration of 1 mM or 5 mM. The results of Examples 6 and 7 show that the samples show melanin production inhibition activity and are low in cytotoxicity.

### Example 8

### (Tyrosinase Activity Inhibition Test)

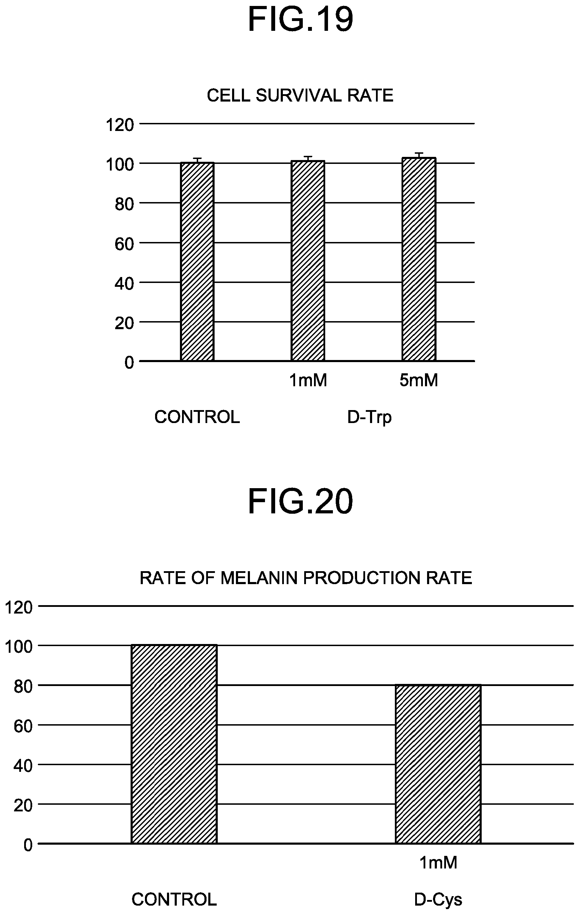

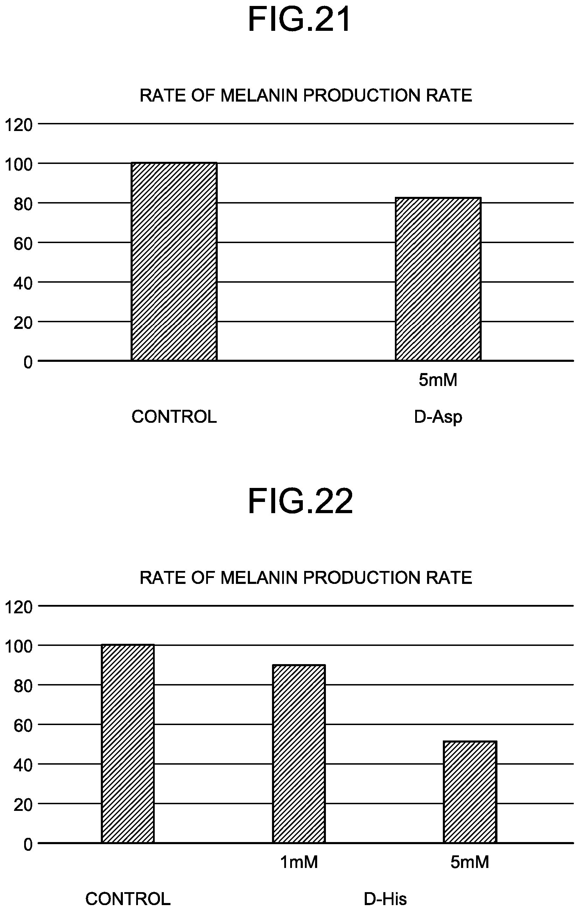

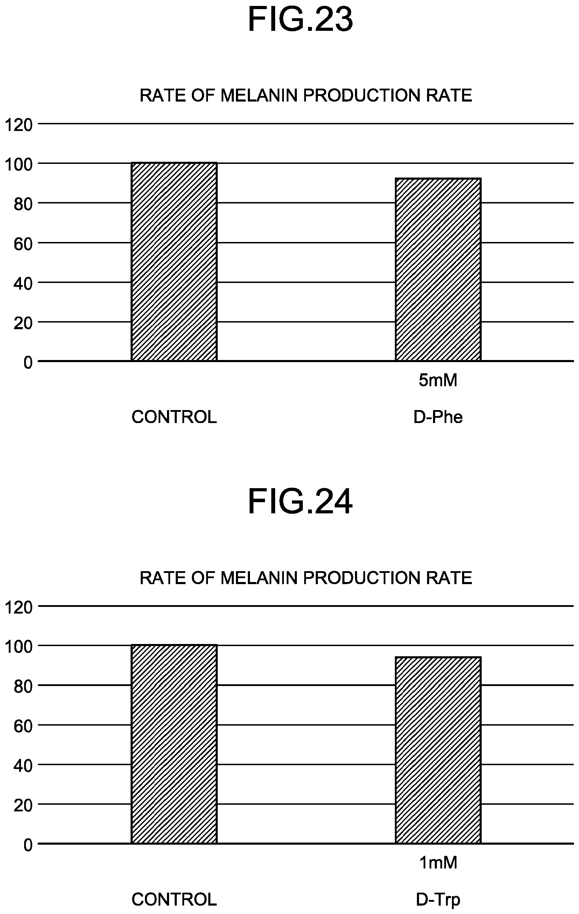

Normal human melanocytes were cultured with a culture medium obtained by adding HMGS custom-made multiplication additive set (Kurabo Industries Ltd.) to Medium 254 (manufactured by Thermo Fisher Scientific). Cells that have become confluent were peeled off with trypsin and were seeded in a 6-well plate. On the next day, after the cells adhered to the plate, culture medium exchange with a culture medium with Phorbol 12-myristate 13-acetate (PMA) removed from the culture medium described above was performed. After 48 hours, exchange with a culture medium with each evaluation sample (a control (without sample addition) and the D-amino acid) added with a certain concentration (1 mM or 5 mM) and with PMA removed was performed, which was cultured for 3 days. The culture medium was removed and was rinsed with PBS diluted 15-fold. A dissolved liquid (1 wt% Triton X-100, PBS diluted 15-fold) was added with 200 µl/well and was shaken with a plate shaker for 5 minutes. The dissolved liquid with cells dissolved by pipetting and stirred was collected to a microtube, and 20 µl thereof was dispensed for protein quantification. The residual dissolved liquid was centrifuged (9,100 g, 4°C, 5 minutes), and 40 µl of a supernatant was added to a 96-well plate. To this 96-well plate, an L-DOPA solution (2 mg/ml, PBS diluted 15-fold) was added with 100 µl/well, for which absorbance at 490 nm was measured every 1 minute while being heated at 37°C. During measurement, the 96-well plate was regularly stirred with a plate reader. From the measured absorbance, a maximum melanin production rate (a change in absorbance per unit time) was calculated, and the maximum melanin production rate of each D-amino acid-added cell with a certain concentration when the maximum melanin production rate of the control (without sample) was defined as 100% was represented as relative percentage, whereby a rate of a melanin production rate of each D-amino acid was calculated. FIGS. 20 to 24 illustrate results.

It can be seen from FIGS. 20 to 24 that the melanin production rate of the cells reduces by the addition of each sample.

### Example 9

### (Protein Quantification)

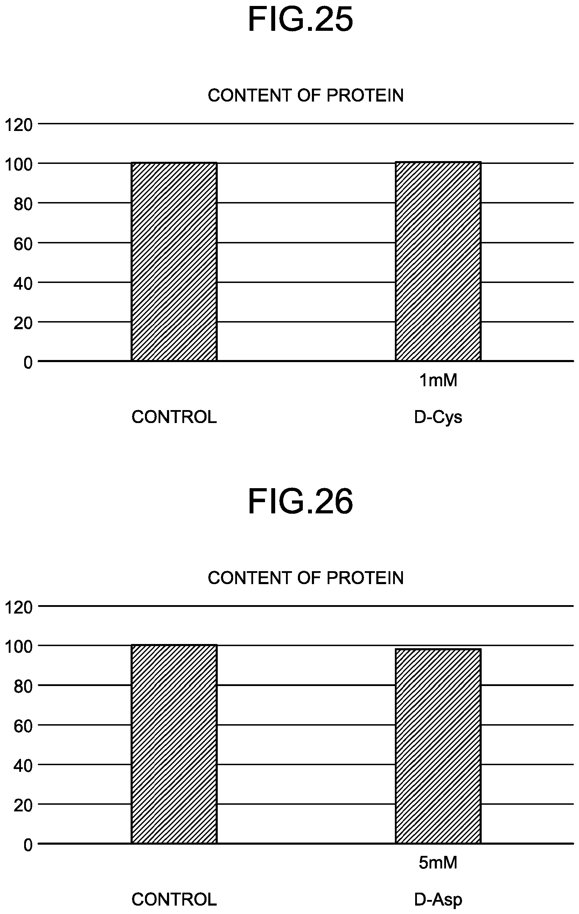

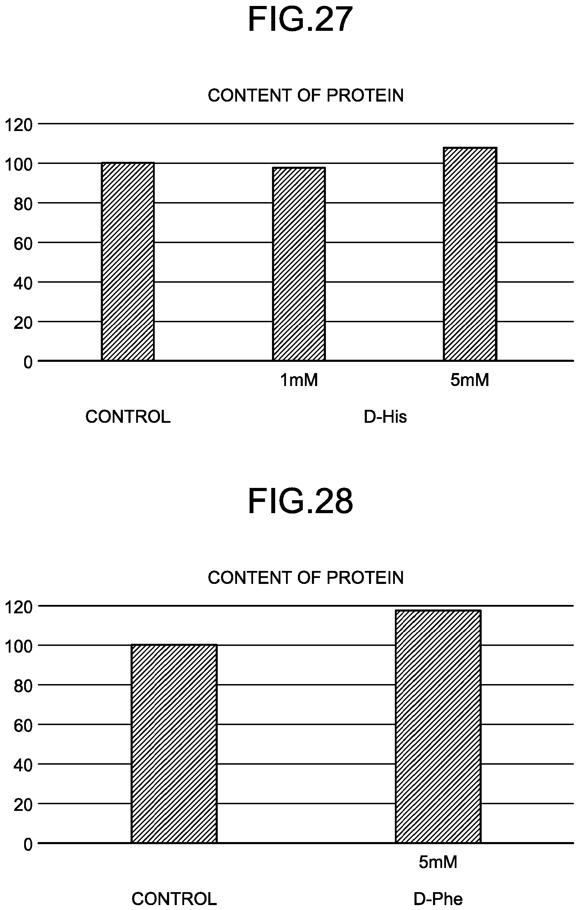

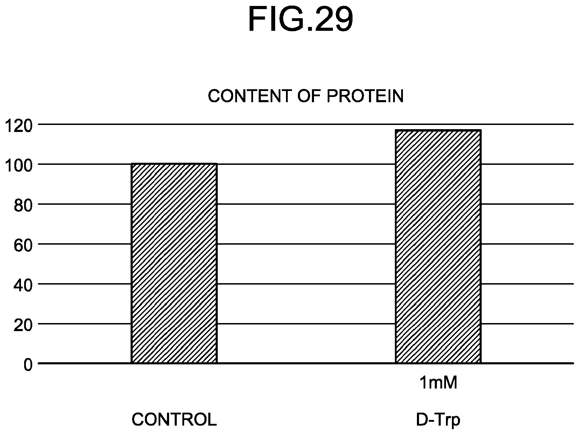

To the cell dissolved liquid (20 µl) dispensed in the tyrosinase activity inhibition test of Example 8, MilliQ water (80 µl) was added to be 100 µl. A content of protein in this solution was quantified using DC protein assay (manufactured by Bio-Rad Laboratories, Inc.). Measurement was performed with 100 µl of Liquid A and 800 µl of Liquid B mixed with 100 µl of a sample solution. The relative percentage of the content of protein with each D-amino acid with a certain centration added when the content of protein of a control (without sample) was defined as 100% was calculated. FIGS. 25 to 29 illustrate results.

From FIGS. 25 to 29, the contents of protein of the respective samples were all 95% or more. The results of Examples 8 and 9 show that the samples have a tyrosinase activity inhibition effect and are low in cytotoxicity.

### (Prescription Example 1; essence)

**[Table 5]**

| Name of component | Amount (parts by mass) | Name of component | Amount (parts by mass) |
|---|---|---|---|
| Lysolecithin | 0.50 | D-Tyr | 0.50 |
| Polyoxyethylene sorbitan monooleate (2 E.O.) | 0.30 | Kojic acid | 1.00 |
| Polyoxyethylene hydrogenated castor oil (40 E.O.) | 0.50 | Trioleyl phosphate | 7.00 |
| Ethanol | 7.00 | 1,3-Butylene glycol | 3.00 |
| Methylphenylpolysiloxane | 3.00 | Polyethylene glycol 1000 | 1.00 |
| Perfume | 0.01 | Potassium hydroxide | 0.15 |
| D-Asn | 0.50 | Lavender extract | 0.01 |
| D-Val | 0.50 | Peppermint extract | 0.01 |
| D-allo-Thr | 0.50 | Sage extract | 0.01 |
| D-Leu | 0.50 | Anise extract | 0.01 |
| D-Gln | 0.50 | Rose extract | 0.01 |
| D-His | 0.50 | Chamaecyparis obtusa water | 0.01 |
| D-Leu | 0.50 | Rooibos extract | 0.01 |
| D-Phe | 0.50 | Lavender extract | 0.10 |
| D-Pro | 0.50 | 10% Aqueous arginine solution | Proper amount |
| D-Ser | 0.50 | Purified water | Remainder |
| D-Trp | 0.50 | | |

### (Prescription Example 2; cream)

**[Table 6]**

| Name of component | Amount (parts by mass) | Name of component | Amount (parts by mass) |
|---|---|---|---|
| Squalane | 8.00 | D-Val | 0.01 |
| Cetyl ethylhexanoate | 3.00 | D-allo-Thr | 0.01 |
| Cetanol | 2.80 | D-Lys | 0.01 |
| Stearic acid | 2.40 | D-Gln | 0.01 |
| PG stearate | 1.20 | D-His | 0.01 |
| Glyceryl stearate (SE) | 3.30 | D-Leu | 0.01 |
| Polysorbate 60 | 0.50 | D-Phe | 0.01 |
| PEG-40 stearate | 1.50 | D-Pro | 0.01 |
| Tocopherol | 0.05 | D-Ser | 0.01 |
| Phytosteryl/decyltetradecyl myristoyl methyl-β-alanine | 1.00 | D-Trp | 0.01 |
| | | D-Tyr | 0.01 |
| Methylparaben | 0.20 | Lavender extract | 0.10 |
| BG | 5.00 | 10% Aqueous L-Arg solution | Proper amount |
| Xanthan gum | 0.10 | Perfume | Proper amount |
| Gellan gum | 0.10 | Purified water | Remainder |
| D-Asn | 0.01 | | |

### (Prescription Example 3; milky lotion)

**[Table 7]**

| Name of component | Amount (parts by mass) | Name of component | Amount (parts by mass) |
|---|---|---|---|
| Stearic acid | 2.00 | D-Ser | 0.50 |
| Cetanol | 1.00 | D-Trp | 0.50 |
| Vaseline | 2.00 | D-Tyr | 0.50 |
| Liquid paraffin | 9.00 | L-Asn | 0.01 |
| Cetyl ethylhexanoate | 1.00 | L-Val | 0.01 |
| Jojoba oil | 1.00 | L-allo-Thr | 0.01 |
| Squalane | 2.00 | L-Lys | 0.01 |
| POE (30) hydrogenated castor oil | 3.00 | Acetylglutamine | 0.01 |
| Methylphenylpolysiloxane | 1.00 | L-His | 0.01 |
| Tocopherol | 0.30 | L-Leu | 0.01 |
| POE (10) monooleate | 0.10 | L-Phe | 0.01 |
| Butylparaben | 0.20 | L-Pro | 0.01 |
| PG | 5.00 | L-Ser | 0.01 |
| BG | 2.00 | L-Trp | 0.01 |
| Carboxyvinyl polymer | 0.50 | L-Tyr | 0.01 |
| Kojic acid | 1.00 | Pyridoxylserine | 0.01 |
| D-Asn | 0.50 | Carnosine | 0.01 |
| D-Val | 0.50 | Acetylmethionine | 0.01 |
| D-allo-Thr | 0.50 | Acetylcysteine | 0.01 |
| D-Lys | 0.50 | Lavender extract | 0.10 |
| D-Gln | 0.50 | EDTA-2Na | 0.05 |
| D-His | 0.50 | Etidronic acid | 0.05 |
| D-Leu | 0.50 | 10% Aqueous L-Arg solution | Proper amount |
| D-Phe | 0.50 | Perfume | Proper amount |
| D-Pro | 0.50 | Purified water | Remainder |

### (Prescription Example 4; rouge)

**[Table 8]**

| Name of component | Amount (parts by mass) | Name of component | Amount (parts by mass) |
|---|---|---|---|
| Candelilla wax | 8.00 | D-Asn | 0.25 |
| Paraffin | 6.00 | D-Val | 0.25 |
| Beeswax | 4.00 | D-allo-Thr | 0.25 |
| Carnauba wax | 2.00 | D-Lys | 0.25 |
| Lanolin | 7.17 | D-Gln | 0.25 |
| Castor oil | 13.30 | D-His | 0.25 |
| Cetyl ethylhexanoate | 13.03 | D-Leu | 0.25 |
| Ethylhexyl palmitate | 10.32 | D-Phe | 0.25 |
| Hydrogenated polyisobutene | 5.00 | D-Pro | 0.25 |
| Red 202 | 1.05 | D-Ser | 0.25 |
| Yellow 4 | 0.90 | D-Trp | 0.25 |
| Blue 1 | 0.04 | D-Tyr | 0.25 |
| Titanium oxide | 1.60 | Dimer dilinoleyl bis(N-lauroyl-L-glutamate/N-lauroyl sarcosinate) | 20.00 |
| Diisostearyl malate | 3.59 | | |
| Mica | 1.00 | | |

### (Prescription Example 5; ointment)

**[Table 9]**

| Name of component | Amount (parts by mass) | Name of component | Amount (parts by mass) |
|---|---|---|---|
| Octyl p-dimethylaminobenzoate | 4.00 | D, L-Leu | 1.00 |
| Butyl methoxybenzoylmethane | 0.50 | D, L-Phe | 1.00 |
| Tocopherol acetate | 0.50 | D, L-Pro | 1.00 |
| Retinol palmitate | 1.00 | D, L-Ser | 1.00 |
| Stearyl alcohol | 18.00 | D, L-Trp | 1.00 |
| Japan wax | 20.00 | D, L-Tyr | 1.00 |
| D, L-Asn | 1.00 | Lavender extract | 0.10 |
| D,L-Val | 1.00 | Polyoxyethylene (10) monooleate | 0.25 |
| D,L-allo-Thr | 1.00 | | |
| D,L-Lys | 1.00 | Glycerin monostearate | 0.30 |
| D, L-Gln | 1.00 | | |
| D, L-His | 1.00 | Vaseline | 43.35 |

### (Prescription Example 6; sunscreen)

**[Table 10]**

| Name of component | Amount (parts by mass) | Name of component | Amount (parts by mass) |
|---|---|---|---|
| Dimethylpolysiloxane | 5.00 | D-His | 0.01 |
| Decamethylcyclopentasiloxane | 25.00 | D-Leu | 0.01 |
| Trimethylsiloxysilicate | 5.00 | D-Phe | 0.01 |
| Polyoxyethylene/methylpolysiloxane copolymer | 2.00 | D-Pro | 0.01 |
| 2-Ethylhexyl p-methoxycinnamate | 7.50 | D-Ser | 0.01 |
| Spherical alkyl polyacrylate powder | 5.00 | D-Trp | 0.01 |
| Dextrin palmitate-coated fine particle zinc oxide (60 nm) | 5.00 | D-Tyr | 0.01 |
| | | Thiotaurine | 0.05 |
| DPG | 5.00 | Sophora angustifolia root extract | 1.00 |
| Dimethyldistearylammonium hectorite | 0.50 | Lavender extract | 0.10 |
| | | Methylparaben | Proper amount |
| D-Asn | 0.01 | Phenoxy ethanol | Proper amount |
| D-Val | 0.01 | Trisodium edetate | Proper amount |
| D-allo-Thr | 0.01 | Butylethyl propanediol | 0.50 |
| D-Lys | 0.01 | Purified water | Remainder |
| D-Gln | 0.01 | Perfume | Proper amount |

### (Prescription Example 7; skin lotion)

**[Table 11]**

| Name of component | Amount (parts by mass) | Name of component | Amount (parts by mass) |
|---|---|---|---|
| Sorbitol | 4.00 | Carnosine | 0.01 |
| DPG | 6.00 | Acetylmethionine | 0.01 |
| PEG-30 | 5.00 | Acetylcysteine | 0.01 |
| POE (20) oleyl alcohol ether | 0.50 | D-Asn | 0.03 |
| | | D-Val | 0.03 |
| Methylcellulose | 0.20 | D-allo-Thr | 0.03 |
| Pyrus cydonia seed | 0.10 | D-Lys | 0.03 |
| Ethanol | 10.00 | D-Gln | 0.03 |
| L-Asn | 0.01 | D-His | 0.03 |
| L-Val | 0.01 | D-Leu | 0.03 |
| L-allo-Thr | 0.01 | D-Phe | 0.03 |
| L-Lys hydrochloride | 0.01 | D-Pro | 0.03 |
| Acetylglutamine | 0.01 | D-Ser | 0.03 |
| L-His | 0.01 | D-Trp | 0.03 |
| L-Leu | 0.01 | D-Tyr | 0.03 |
| L-Phe | 0.01 | Lavender extract | 0.10 |
| L-Pro | 0.01 | EDTA-2Na | 0.05 |
| L-Ser | 0.01 | Etidronic acid | 0.05 |
| L-Trp | 0.01 | Tocopherol | 0.30 |
| L-Tyr | 0.01 | 10% Aqueous L-Arg solution | Proper amount |
| Sodium L-Glu | 0.01 | Perfume | Proper amount |
| Gly | 0.01 | Purified water | Remainder |
| Pyridoxylserine | 0.01 | | |

### (Prescription Example 8; jelly pack)

**[Table 12]**

| Name of component | Amount (parts by mass) | Name of component | Amount (parts by mass) |
|---|---|---|---|
| Glycerin | 10 | Betaine | 0.05 |
| Diglycerin | 3 | D-Amino acid | 12 |
| BG | 3 | Arbutin | 0.10 |
| DPG | 1 | Panthenol | 0.10 |
| PEG-30 | 0.5 | Niacinamide | 0.10 |
| Honey | 2 | Pyridoxine HCl | 0.10 |
| Xanthan gum | 0.1 | Retinol | 0.10 |
| Carrageenan | 0.5 | Carotene | 0.10 |
| Pectin | 0.5 | Riboflavin | 0.10 |
| Jojoba oil | 5 | Tocopherol | 0.10 |
| Neopentyl glycol dicaprate | 5 | Ubiquinone | 0.10 |
| Dimethicone | 0.1 | Sodium hyaluronate | 0.10 |
| Cyclohexasiloxane | 0.1 | Water-soluble collagen | 0.10 |
| Cyclopentasiloxane | 0.1 | Sodium chondroitin sulfate | 0.10 |
| Phytosteryl/decyltetradecyl myristoyl methyl-β-alanine | 0.2 | Lactobacillus/Milk ferment filtrate | 5.00 |
| Isopropyl lauroyl sarcosinate | 0.2 | Whey | 1.00 |
| Lauroyl lysine | 0.1 | Yogurt extract | 1.00 |
| Talc surface-treated with lauroyl lysine | 0.1 | Sodium hydrolyzed casein | 0.10 |
| Ethylhexyl dimethoxybenzylidene dioxoimidazolidine propionate | 0.1 | Soybean ferment extract | 0.10 |
| Dibutyl ethylhexanoyl glutamide | 0.1 | Lavender extract | 0.10 |
| Dibutyl lauroyl glutamide | 0.1 | Benzalkonium chloride | 0.10 |
| Bentonite | 0.1 | Triclosan | 0.10 |
| Polysorbate 65 | 2.5 | Salicylic acid | 0.10 |
| L-Amino acid | 1.35 | Citric acid | 0.10 |
| Acetylglutamine | 0.05 | Bisabolol | 0.10 |
| Acetylcysteine | 0.05 | Calcium chloride | 0.10 |
| Sodium polyaspartate | 0.05 | EDTA-2Na | 0.10 |
| Pyridoxylserine | 0.05 | Glyceryl caprylate | 0.10 |
| Taurine | 0.05 | Phenoxy ethanol | 0.30 |
| Carnosine | 0.05 | Ethanol | 0.50 |
| Citrulline | 0.05 | Perfume | Proper amount |
| Ornithine | 0.05 | Purified water | Remainder |

Table 13 lists the details of D-amino acid and L-amino acid in Table 12.

**[Table 13]**

| L-Amino acid | Amount (parts by mass) | D-Amino acid | Amount (parts by mass) |
|---|---|---|---|
| L-Asn | 0.05 | D-Asn | 1.00 |
| L-Val | 0.05 | D-Val | 1.00 |
| L-Thr | 0.05 | D-allo-Thr | 1.00 |
| L-Lys | 0.05 | D-Lys | 1.00 |
| L-His | 0.05 | D-Gln | 1.00 |
| L-Leu | 0.05 | D-His | 1.00 |
| L-Phe | 0.05 | D-Leu | 1.00 |
| L-Pro | 0.05 | D-Phe | 1.00 |
| L-Ser | 0.05 | D-Pro | 1.00 |
| L-Trp | 0.05 | D-Ser | 1.00 |
| L-Tyr | 0.05 | D-Trp | 1.00 |
| L-Arg | 0.50 | D-Tyr | 1.00 |
| L-Glu | 0.05 | | |
| L-Asp | 0.05 | | |
| L-Ile | 0.05 | | |
| Gly | 0.05 | | |
| L-Ala | 0.05 | | |
| L-Met | 0.05 | | |

### (Prescription Example 9; o/w type foundation)

**[Table 14]**

| Name of component | Amount (parts by mass) | Name of component | Amount (parts by mass) |
|---|---|---|---|
| Talc | 3.00 | Acetylglutamine | 0.01 |
| Titanium oxide | 5.00 | L-His | 0.01 |
| Bengala | 0.50 | L-Phe | 0.01 |
| Yellow iron oxide | 1.40 | L-Pro | 0.01 |
| Black iron oxide | 0.10 | L-Ser | 0.01 |
| Bentonite | 0.50 | L-Trp | 0.01 |
| Polysorbate 60 | 0.90 | L-Ile | 0.01 |
| Triethanolamine | 1.00 | L-Asp | 0.01 |
| PG | 10.00 | Sodium L-Glu | 0.01 |
| Kojic acid | 1.00 | L-Arg | 0.01 |
| D-Asn | 0.02 | L-Ala | 0.01 |
| D-Val | 0.02 | Gly | 0.01 |
| D-allo-Thr | 0.02 | Pyridoxylserine | 0.01 |
| D-Lys | 0.02 | Carnosine | 0.01 |
| D-Gln | 0.02 | Acetylmethionine | 0.01 |
| D-His | 0.02 | Acetylcysteine | 0.01 |
| D-Leu | 0.02 | Lavender extract | 0.10 |
| D-Phe | 0.02 | Stearic acid | 2.20 |
| D-Pro | 0.02 | Isohexadecyl alcohol | 7.00 |
| D-Ser | 0.02 | Glyceryl stearate | 2.00 |
| D-Trp | 0.02 | Liquid lanolin | 2.00 |
| D-Tyr | 0.02 | Liquid paraffin | 8.00 |
| L-Val | 0.01 | 10% Aqueous L-Arg solution | Proper amount |
| L-Thr | 0.01 | Methylparaben | 0.20 |
| L-Lys hydrochloride | 0.01 | Perfume | Proper amount |
| | | Purified water | Remainder |

### (Prescription Example 10; makeup cleansing)

**[Table 15]**

| Name of component | Amount (parts by mass) | Name of component | Amount (parts by mass) |
|---|---|---|---|
| Mineral oil | 38.00 | D-Lys | 0.01 |
| Octyl palmitate | 20.00 | D-Gln | 0.01 |
| Triethylhexanoin | 10.00 | D-His | 0.01 |
| Isopropyl palmitate | 10.00 | D-Leu | 0.01 |
| Octyldodecyl myristate | 10.00 | D-Phe | 0.01 |
| PEG-20 glyceryl triisostearate | 10.00 | D-Pro | 0.01 |
| | | D-Ser | 0.01 |
| D-Asn | 0.01 | D-Trp | 0.01 |
| D-Val | 0.01 | D-Tyr | 0.01 |
| D-allo-Thr | 0.01 | Purified water | Remainder |

### (Prescription Example 11; cleansing foam)

**[Table 16]**

| Name of component | Amount (parts by mass) | Name of component | Amount (parts by mass) |
|---|---|---|---|
| Sodium lauroyl glutamate | 35.00 | D-His | 0.10 |
| Potassium myristate | 5.00 | D-Leu | 0.10 |
| Cocamide MEA | 1.00 | D-Phe | 0.10 |
| BG | 10.00 | D-Pro | 0.10 |
| Dipropylene glycol | 20.00 | D-Ser | 0.10 |
| PVP | 0.30 | D-Trp | 0.10 |
| D-Asn | 0.10 | D-Tyr | 0.10 |
| D-Val | 0.10 | Talc | 1.00 |
| D-allo-Thr | 0.10 | Perfume | Proper amount |
| D-Lys | 0.10 | Purified water | Remainder |
| D-Gln | 0.10 | | |

### (Prescription Example 12; body shampoo)

**[Table 17]**

| Name of component | Amount (parts by mass) | Name of component | Amount (parts by mass) |
|---|---|---|---|
| Sodium laureth sulfate | 3.00 | D-Asn | 0.01 |
| Cocamidopropyl betaine | 2.00 | D-Val | 0.01 |
| Lauric acid | 6.52 | D-allo-Thr | 0.01 |
| Myristic acid | 5.25 | D-Lys | 0.01 |
| Palmitic acid | 3.90 | D-G In | 0.10 |
| Stearic acid | 0.80 | D-His | 0.10 |
| Glycol distearate | 2.50 | D-Leu | 0.01 |
| PG | 3.50 | D-Phe | 0.01 |
| Cocamide MEA | 0.50 | D-Pro | 0.01 |
| Cocamide methyl MEA | 1.50 | D-Ser | 0.01 |
| Sodium cocoamphoacetate | 0.20 | D-Trp | 0.01 |
| Hydroxypropyl methylcellulose | 0.20 | D-Tyr | 0.01 |
| Polyquaternium-7 | 0.10 | Potassium hydroxide | 2.00 |
| PEG-7M | 0.05 | Methylparaben | 0.20 |
| Guar hydroxypropyltrimonium chloride | 0.05 | EDTA-2Na | 0.10 |
| | | Purified water | Remainder |

## Claims

1. Non-therapeutic use of a composition comprising at least one D-amino acid selected from the group consisting of D-asparagine, D-valine, D-allo-threonine, D-lysine, D-glutamine, D-histidine, D-phenylalanine, and D-tryptophan, as a skin-whitening agent.

2. The non-therapeutic use according to claim 1, wherein the D-amino acid is at least one selected from the group consisting of D-asparagine, D-allo-threonine, D-lysine, D-histidine, D-phenylalanine, and D-tryptophan.

3. The non-therapeutic use according to claim 1, wherein the D-amino acid is at least one selected from the group consisting of D-allo-threonine, D-lysine, D-histidine, D-phenylalanine, and D-tryptophan.

4. The non-therapeutic use according to claim 1, wherein
the skin-whitening agent is a production inhibitor of at least one melanin production-related protein selected from the group consisting of tyrosinase, tyrosinase-related protein 1, and melanocortin 1 receptor, and
the at least one D-amino acid is selected from the group consisting of D-asparagine, D-phenylalanine, D-tryptophan, and D-histidine.

5. Non-therapeutic use of a composition comprising at least one D-amino acid selected from the group consisting of D-asparagine, D-valine, D-allo-threonine, D-lysine, D-glutamine, D-histidine, D-phenylalanine, and D-tryptophan for the prevention, inhibition, or treatment of pigmented spot, freckles, or somberness.

6. A method of evaluation of a skin by a D-amino acid, the method comprising:
measuring at least either a melanin index or an L* value of a skin of a subject to determine an appearance of the skin of the subject;
collecting a horny layer from a pigmentation site of the skin of the subject;
identifying at least one D-amino acid level contained in the horny layer; and
comparing a relation between the appearance of the skin and the D-amino acid level among results of the subject.

7. The method of evaluation according to claim 6, wherein the identification of the D-amino acid level is quantification of a content of the D-amino acid per content of protein contained in the horny layer.

8. The method of evaluation according to claim 6 or 7, wherein the quantification of the content of the D-amino acid is performed with a liquid chromatograph-tandem mass spectrometer.

9. The method of evaluation according to claim 7 or 8, wherein measurement of the content of protein is performed by the BCA method.

10. The method of evaluation according to any one of claims 6 to 9, wherein the D-amino acid comprises at least one selected from the group consisting of D-asparagine, D-valine, D-allo-threonine, D-lysine, D-glutamine, D-histidine, D-phenylalanine, D-proline, and D-tryptophan.

11. The method of evaluation according to claim 10, wherein
the subject is a woman in her twenties or thirties, and
the D-amino acid is at least one selected from the group consisting of D-lysine, D-glutamine, D-histidine, D-proline, and D-tryptophan.

12. The method of evaluation according to claim 10, wherein
the subject is a woman in her forties or fifties, and
the D-amino acid is at least one selected from the group consisting of D-asparagine, D-valine, D-allo-threonine, D-lysine, D-glutamine, D-histidine, D-phenylalanine, and D-tryptophan.

## Patentansprüche

1. Nichttherapeutische Verwendung einer Zusammensetzung, die zumindest eine D-Aminosäure umfasst, die aus der aus D-Asparagin, D-Valin, D-allo-Threonin, D-Lysin, D-Glutamin, D-Histidin, D-Phenylalanin und D-Tryptophan bestehenden Gruppe ausgewählt ist, als Hautaufhellungsmittel.

2. Nichttherapeutische Verwendung nach Anspruch 1, wobei die D-Aminosäure zumindest eine ist, die aus der aus D-Asparagin, D-allo-Threonin, D-Lysin, D-Histidin, D-Phenylalanin und D-Tryptophan bestehenden Gruppe ausgewählt ist.

3. Nichttherapeutische Verwendung nach Anspruch 1, wobei die D-Aminosäure zumindest eine ist, die aus der aus D-allo-Threonin, D-Lysin, D-Histidin, D-Phenylalanin und D-Tryptophan bestehenden Gruppe ausgewählt ist.

4. Nichttherapeutische Verwendung nach Anspruch 1, wobei das Hautaufhellungsmittel ein Produktionsinhibitor von zumindest einem die Melaninproduktion betreffenden Protein ist, das aus der aus Tyrosinase, Tyrosinase-verwandtem Protein 1 und dem Melanocortin-1-Rezeptor bestehenden Gruppe ausgewählt ist, und
die zumindest eine D-Aminosäure aus der aus D-Asparagin, D-Phenylalanin, D-Tryptophan und D-Histidin bestehenden Gruppe ausgewählt ist.

5. Nichttherapeutische Verwendung einer Zusammensetzung, umfassend zumindest eine D-Aminosäure, die aus der aus D-Asparagin, D-Valin, D-allo-Threonin, D-Lysin, D-Glutamin, D-Histidin, D-Phenylalanin und D-Tryptophan bestehenden Gruppe ausgewählt ist, zur Prävention, Inhibierung oder Behandlung von Pigmentflecken, Sommersprossen oder dunklen Stellen.

6. Verfahren zur Bewertung einer Haut durch D-Aminosäure, wobei das Verfahren Folgendes umfasst:
Messen von zumindest entweder einem Melaninindex oder einem L*-Wert einer Haut eines Individuums, um ein Erscheinungsbild der Haut des Individuums zu bestimmen;
Entnehmen einer Hornhautschicht von einer Pigmentierungsstelle der Haut des Individuums;
Identifizieren der Menge von zumindest einer D-Aminosäure, die in der Hornhautschicht enthalten ist; und
Vergleichen einer Beziehung zwischen dem Erscheinungsbild der Haut und dem Wert der D-Aminosäure aus Ergebnissen des Individuums.

7. Verfahren zur Bewertung nach Anspruch 6, wobei die Identifikation der Menge der D-Aminosäure die Quantifizierung eines Gehalts der D-Aminosäure pro Gehalt von Protein, das in der Hornhautschicht enthalten ist, ist.

8. Verfahren zur Bewertung nach Anspruch 6 oder 7, wobei die Quantifizierung des Gehalts der D-Aminosäure mit einem Flüssigchromatographie-Tandem-Massenspektrometer durchgeführt wird.

9. Verfahren zur Bewertung nach Anspruch 7 oder 8, wobei die Messung des Gehalts des Proteins durch das BCA-Verfahren durchgeführt wird.

10. Verfahren zur Bewertung nach einem der Ansprüche 6 bis 9, wobei die D-Aminosäure zumindest eine umfasst, die aus der aus D-Asparagin, D-Valin, D-allo-Threonin, D-Lysin, D-Glutamin, D-Histidin, D-Phenylalanin, D-Prolin und D-Tryptophan bestehenden Gruppe ausgewählt ist.

11. Verfahren zur Bewertung nach Anspruch 10, wobei
das Individuum eine Frau in den Zwanzigern oder Dreißigern ist und
die D-Aminosäure zumindest eine ist, die aus der aus D-Lysin, D-Glutamin, D-Histidin, D-Prolin und D-Tryptophan bestehenden Gruppe ausgewählt ist.

12. Verfahren zur Bewertung nach Anspruch 10, wobei
das Individuum eine Frau in den Vierzigern oder Fünfzigern ist und
die D-Aminosäure zumindest eine ist, die aus der aus D-Asparagin, D-Valin, D-allo-Threonin, D-Lysin, D-Glutamin, D-Histidin, D-Phenylalanin und D-Tryptophan bestehenden Gruppe ausgewählt ist.

## Revendications

1. Utilisation non thérapeutique d'une composition comprenant au moins un acide D-aminé choisi dans le groupe constitué de la D-asparagine, de la D-valine, de la D-allo-thréonine, de la D-lysine, de la D-glutamine, de la D-histidine, de la D-phénylalanine, comme agent blanchissant la peau.

2. Utilisation non thérapeutique selon la revendication 1, dans laquelle l'acide D-aminé est au moins un choisi dans le groupe constitué de la D-asparagine, de la D-allo-thréonine, de la D-lysine, de la D-histidine, de la D-phénylalanine et du D-tryptophane.

3. Utilisation non thérapeutique selon la revendication 1, dans laquelle l'acide D-aminé est au moins un acide sélectionné dans le groupe constitué de la D-allo-thréonine, de la D-lysine, de la D-histidine, de la D-phénylalanine et du D-tryptophane.

4. Utilisation non thérapeutique selon la revendication 1, dans laquelle
l'agent de blanchiment de la peau est un inhibiteur de production d'au moins une protéine liée à la production de mélanine sélectionnée dans le groupe constitué de la tyrosinase, de la protéine 1 liée à la tyrosinase et du récepteur de mélanocortine 1, et
ledit au moins un acide D-aminé est choisi dans le groupe constitué de la D-asparagine, de la D-phénylalanine, du D-tryptophane et de la D-histidine.

5. Utilisation non thérapeutique d'une composition comprenant au moins un acide D-aminé choisi dans le groupe constitué de la D-asparagine, de la D-valine, de la D-allo-thréonine, de la D-lysine, de la D-glutamine, de la D-histidine, de la D-phénylalanine et du D-tryptophane pour la prévention, l'inhibition ou le traitement des taches pigmentées, des taches de rousseur ou des taches sombres.

6. Procédé d'évaluation d'une peau par l'intermédiaire d'un acide D-aminé, le procédé comprenant les étapes consistant à :
mesurer au moins soit un indice de mélanine, soit une valeur L* d'une peau d'un sujet pour déterminer un aspect de la peau du sujet ;
collecter une couche cornée à partir d'un site de pigmentation de la peau du sujet ;
identifier au moins un niveau d'acide D-aminé contenu dans la couche cornée ; et
comparer une relation entre l'apparence de la peau et le niveau d'acide D-aminé parmi des résultats du sujet.

7. Procédé d'évaluation selon la revendication 6, dans lequel l'identification du niveau d'acide D-aminé est la quantification d'une teneur d'acide D-aminé par une teneur de protéine contenue dans la couche cornée.

8. Procédé d'évaluation selon la revendication 6 ou 7, dans lequel la quantification de la teneur de l'acide D-aminé est effectuée avec un spectromètre de masse à chromatographe liquide en tandem.

9. Procédé d'évaluation selon la revendication 7 ou 8, dans lequel la mesure de la teneur de protéine est effectuée par le procédé BCA.

10. Procédé d'évaluation selon l'une quelconque des revendications 6 à 9, dans lequel l'acide D-aminé comprend au moins un acide sélectionné dans le groupe constitué de la D-asparagine, de la D-valine, de la D-allo-thréonine, de la D-lysine, de la D-glutamine, de la D-histidine, de la D-phénylalanine, de la D-proline et du D-tryptophane.

11. Procédé d'évaluation selon la revendication 10, dans lequel
le sujet est une femme dans la vingtaine ou la trentaine, et
l'acide D-aminé est au moins un sélectionné dans le groupe constitué de la D-lysine, de la D-glutamine, de la D-histidine, de la D-praline et du D-tryptophane.

12. Procédé d'évaluation selon la revendication 10, dans lequel
le sujet est une femme dans la quarantaine ou la cinquantaine, et
l'acide D-aminé est au moins un sélectionné dans le groupe constitué de la D-asparagine, de la D-valine, de la D-allo-thréonine, de la D-lysine, de la D-glutamine, de la D-histidine, de la D-phénylalanine et du D-tryptophane.
